# EUROPÄISCHE PATENTSCHRIFT

(11) **EP 1 340 746 B1**
(45) Veröffentlichungstag und Bekanntmachung des Hinweises auf die Patenterteilung: **28.02.2007**
(21) Anmeldenummer: 03003254.4
(22) Anmeldetag: 24.02.2003
(51) Int. Cl.: C07D 213/55, C07D 213/56, C07D 213/61, C07D 333/24, C07D 333/60, C07D 333/62, C07D 307/54

(54) **Verfahren zur Reduktion von 3-Heteroaryl-3-Oxopropionsäurederivaten**
Process for the reduction of 3-heteroaryl-3-oxo-propanoic acid derivatives
Procédé pour la réduction de dérivés de l'acide 3-hétéroaryl-3-oxo-propanoique

(30) Priorität: 01.03.2002 DE 10208828
(43) Veröffentlichungstag der Anmeldung: 03.09.2003
(73) Patentinhaber: Saltigo GmbH, 51369 Leverkusen (DE)
(72) Erfinder: Bosch, Boris, Elmar, Dr., 50668 Köln (DE); Eckert, Markus, Dr., CN Shanghai 200001 (CN); Militzer, Hans-Christian, Dr., 51519 Odenthal (DE); Dreisbach, Claus, Dr., 42799 Leichlingen (DE)
(74) Vertreter: Wichmann, Birgid

(56) Entgegenhaltungen:
- MADEC J ET AL: "Asymmetric hydrogenation reactions using a practical in situ generation of chiral ruthenium-diphosphine catalysts from anhydrous RuCl3" TETRAHEDRON, ELSEVIER SCIENCE PUBLISHERS, AMSTERDAM, NL, Bd. 57, Nr. 13, 26. März 2001 (2001-03-26), Seiten 2563-2568, XP004232215 ISSN: 0040-4020
- NOYORI R ET AL: "Ruthenium(II)-Catalyzed Asymmetric Transfer Hydrogenation of Ketones Using a Formic Acid-Triethylamine Mixture" JOURNAL OF THE AMERICAN CHEMICAL SOCIETY, AMERICAN CHEMICAL SOCIETY, WASHINGTON, DC, US, Bd. 118, Nr. 10, 1996, Seiten 2521-2522, XP002240976 ISSN: 0002-7863

## Beschreibung

Die vorliegende Erfindung betrifft ein Verfahren zur Herstellung stereoisomerenangereicherter 3-Heteroaryl-3-hydroxypropionsäurederivaten durch Reduktion von 3-Heteroaryl-3-oxopropionsäurederivaten in Gegenwart von Ruthenium enthaltenden Katalysatoren.

Stereoisomerenangereicherte 3-Hydroxypropionsäurederivate, insbesondere solche, die in 3-Position einen Heteroarylrest tragen, sind wertvolle Zwischenprodukte zum Beispiel bei der Herstellung von flüssigkristallinen Verbindungen, Agrarchemikalien und Pharmazeutika.

Die katalytische Reduktion von Ketonen-zu stereoisomerenangereicherten sekundären Alkoholen ist prinzipiell bekannt. Als Reduktionsmittel kommen dabei üblicherweise molekularer Wasserstoff oder bei sogenannten Transferhydrierungen organische Wasserstoffdonoren wie zum Beispiel Ameisensäure oder Isopropanol in Frage.

Ein Vorteil von Transferhydrierungen besteht darin, dass die sicherheitstechnischen Vorkehrungen, die mit der Handhabung hochentzündlichen molekularen Wasserstoffs unter Druck getroffen werden müssen, entfallen. Weiterhin kann in der Regel bei Umgebungsdruck gearbeitet werden.

Eine zusammenfassende Darstellung über Transferhydrierungen als Methode zur katalytischen Reduktion von Ketonen geben z.B. Zassinovich et al. in Chem. Rev. 1992, 92, 1051-1069 und Noyori et al. in Acc. Chem. Res. 1997, 30, 97-102 und Wills et al. in Tetrahedron, Asymmetry, 1999, 2045.

Noyori et al. (JACS 1996, 118, 2521-2522, Acc. Chem. Res. 1997, 30, 97-102) beschreiben die Verwendung von Rutheniumkomplexen als Katalysatoren und Triethylamin/Ameisensäure für die enantioselektive Reduktion einfacher Ketone. beschreibt ein Verfahren zur stereoisomerenangereicherten Hydrierung von β-Ketoestern mit einem Ruthenium enthaltenden Katalysator zu β-Hydroxyestern, wobei in Tabelle 2 auf S. 2565 die Umsetzung von Methyl-3-oxo-3-(2-thiophenyl)propanoat zu Methyl-3-Hydroxy-3-(2-thiophenyl)-propanoat offenbart ist.

Es bestand jedoch weiterhin das Bedürfnis, ein effizientes Verfahren bereitzustellen, das die Herstellung von stereoisomerenangereicherten 3-Heteroaryl-3-hydroxypropionsäurederivaten aus 3-Heteroaryl-3-oxopropionsäurederivaten erlaubt.

Es wurde nun ein Verfahren zur Herstellung von stereoisomerenangereicherten 3-Heteroaryl-3-hydroxypropionsäurederivaten gefunden, das dadurch gekennzeichnet ist, dass
a) Verbindungen der Formel (I)

   Heteroaryl-CO-CH₂W (I),

   in der
   - Heteroaryl: für einen mono-, bi- oder tricyclischen aromatischen Rest mit insgesamt 5 bis 18 Ringatomen steht, wobei pro Cyclus keines, eines zwei oder drei Ringatome und im gesamten aromatischen Rest ein, zwei, drei, vier oder fünf Ringatome ausgewählt aus der Gruppe Sauerstoff, Schwefel und Stickstoff vorhanden sein können, und wobei der mono-, bi- oder tricyclische aromatische Rest gegebenenfalls einfach oder mehrfach substituiert ist und
   - W: für C(O)YR¹ₙ steht, wobei Y für Sauerstoff steht und n = 1 ist oder Y für Stickstoff steht und n = 2 ist, oder
   - W: für CN steht und
   - R¹: jeweils unabhängig voneinander für Wasserstoff, C₁-C₂₀-Alkyl, C₄-C₁₄-Aryl oder C₅-C₁₅-Arylalkyl oder im Falle, dass Y für Stickstoff steht, die beiden Reste R¹ zusammen für C₃-C₁₂ Alkylen stehen,
b) in Gegenwart eines Ruthenium enthaltenden Katalysators und
c) in Gegenwart von mindestens einem Amin, das zumindest teilweise in protonierter Form vorliegt,
d) mit Ameisensäure, Formiaten oder Mischungen davon
e) gegebenenfalls in Gegenwart von organischem Lösungsmittel
umgesetzt werden.

Es sei darauf hingewiesen, dass vom Umfang der Erfindung auch beliebige Kombinationen der genannten Bereiche und:Vorzugsbereiche für jedes Merkmal umfasst sind.

Stereoisomerenangereicherte (enantiomerenangereicherte, diastereomerenangereicherte) 3-Heteroaryl-3-Hydroxypropionsäurederivate im Sinne der Erfindung bedeuten stereoisomerenreine (enantiomerenreine bzw. diastereomerenreine) 3-Heteroaryl-3-Hydroxypropionsäurederivate oder Mischungen von stereoisomeren (enantiomeren bzw. diastereomeren) 3-Heteroaryl-3-Hydroxypropionsäurederivaten, in denen ein Stereoisomeres (Enantiomeres bzw. Diastereomeres) in einem größeren absoluten Anteil, bevorzugt 70 bis 100 Mol-% und ganz besonders bevorzugt 85 bis 100 Mol-%, vorliegt, als ein anderes Diastereomeres bzw. als das andere Enantiomere.

Alkyl steht im Rahmen der Erfindung jeweils unabhängig für einen geradkettigen oder cyclischen, unabhängig davon verzweigten oder unverzweigten Alkyl-Rest, der durch C₁-C₄-Alkoxy-Reste weiter substituiert sein kann. Gleiches gilt für den Alkylenteil eines Arylalkyl-Restes.

Beispielsweise steht im Rahmen der Erfindung C₁-C₄-Alkyl für Methyl, Ethyl, 2-Ethoxyethyl, n-Propyl, Isopropyl, n-Butyl und tert.-Butyl, C₁-C₈-Alkyl darüber hinaus beispielsweise für n-Pentyl, Cyclohexyl, n-Hexyl, n-Heptyl, n-Octyl oder iso-Octyl, C₁-C₁₂-Alkyl, weiter darüber hinaus z. B. für Norbomyl, n-Decyl und n-Dodecyl und C₁-C₂₀-Alkyl noch weiter darüber hinaus für n-Hexadecyl und n-Octadecyl.

**Aryl** steht im Rahmen der Erfindung beispielsweise und bevorzugt für carbocyclische aromatische Reste oder heteroaromatische Reste, die keines, ein, zwei oder drei Heteroatome pro Cyclus, im gesamten heteroaromatische Rest mindestens jedoch ein Heteroatom enthalten, das ausgewählt aus der Gruppe Stickstoff, Schwefel oder Sauerstoff.

Weiterhin können die carbocyclischen aromatischen Reste oder heteroaromatischen Reste mit bis zu fünf Substituenten pro Cyclus substituiert sein, die jeweils unabhängig voneinander beispielsweise und bevorzugt ausgewählt sind aus der Gruppe Hydroxy, C₁-C₁₂-Akyl, Cyano, COOH, COOM, wobei M für ein Alkalimetallion oder ein halbes Äquivalent eines Erdalkalimetallions steht, COO-(C₁-C₁₂-Akyl), COO-(C₄-C₁₀-Aryl), CO-(C₁-C₁₂-Alkyl), CO-(C₄-C₁₀-Aryl), O-(C₁-C₁₂-Alkyl), O-(C₄-C₁₀-Aryl, N(C₁-C₁₂-Alkyl)₂, NH-(C₁-C₁₂-Alkyl), Fluor, Chlor, Brom, C₁-C₁₂-Fluoralkyl, wobei Fluoralkyl für einen einfach mehrfach oder vollständig durch Fluor substituierten Alkylrest gemäß obiger Definition steht, CONH₂, CONH-(C₁-C₁₂-Alkyl), NHCOO-(C₁-C₁₂-Alkyl). Gleiches gilt für den Arylteil eines Arylalkyl-Restes.

In Formel (I) steht Heteroaryl bevorzugt für einen mono- oder bicyclischen aromatischen Rest mit insgesamt 5 bis 12 Ringatomen, wobei pro Cyclus keines, eines oder zwei und im gesamten aromatischen Rest ein, zwei, drei oder vier Ringatome ausgewählt aus der Gruppe Sauerstoff, Schwefel und Stickstoff vorhanden sein können, und wobei der mono- oder bicyclische aromatische Rest keinen, einen, zwei oder drei Reste pro Cyclus trägt, die jeweils unabhängig voneinander ausgewählt sind aus der Gruppe Hydroxy, C₁-C₁₂-Alkyl, Cyano, COOH, COOM, COO-(C₁-C₁₂-Alkyl), COO-(C₄-C₁₀-Aryl), CO-(C₁-C₁₂-Alkyl), CO-(C₄-C₁₀-Aryl), O-(C₁-C₁₂-Alkyl), (C₁-C₁₂-Alkyl)-O-(C₁-C₁₂-Alkyl), (C₄-C₁₀-Aryl)-O-(C₁-C₁₂-Alkyl), O-(C₄-C₁₀-Aryl), O-CO-(C₄-C₁₀-Aryl), O-CO-(C₁-C₁₂-Alkyl), OCOO-(C₁-C₁₂-Alkyl), N-(C₁-C₁₂-Alkyl)₂; NH-(C₁-C₁₂-Alkyl), N(C₄-C₁₀-Aryl)₂, NH-(C₄-C₁₀-Aryl), Fluor, Chlor, Brom, Iod, NO₂, SO₃H, SO₃M, SO₂(C₁-C₁₂-Alkyl), SO(C₁-C₁₂-Alkyl), C₁-C₁₂-Fluoralkyl, wobei Fluoralkyl für einen einfach, mehrfach oder vollständig durch Fluor substituierten Alkylrest gemäß obiger Definition steht, NHCO-(C₁-C₁₂-Alkyl), CONH₂, CONH-(C₁-C₁₂-Alkyl), NHCOO-(C₁-C₁₂-Alkyl), PO(C₄-C₁₀-Aryl)₂, PO(C₁-C₁₂-Alkyl)₂, PO₃H₂, PO₃M₂, PO₃HM, PO(O(C₁-C₁₂-Alkyl)₂, wobei M jeweils für ein Alkalimetallion oder ein halbes Äquivalent eines Erdalkalimetallions steht.

In Formel (I) steht Heteroaryl besonders bevorzugt für 2- oder 3-Thiophenyl, 2- oder 3-Furanyl, 2- oder 3-Pyrrolyl, 3- oder 4-Pyrazolyl, 1-, 2-, oder 4-Thiazolyl, 1-, 2-, oder 4-Oxazolyl, 2-, 4- oder 5-Imidazolyl, 2-, 3-, oder 4-Pyridyl, 2- oder 3-Pyrazinyl, 2-, 4-, oder 5-Pyrimidyl, 3-, 4-, 5- oder 6-Pyridazinyl, 2- oder 3-Indolyl, 3-Indazolyl, Indazolyl, 2- oder 3-Benzofuranyl, 2- oder 3-Benzothiophen-yl, 2-, 3- oder 4-Chinolinyl, Isochinolinyl 2-, 4-, 6- oder 7-Pteridinyl oder 2- , 3-, 4-, 5-, 6-, 8-, 9- oder 10-Phenanthrenyl wobei jeder der genannten Reste keinen, einen oder zwei Reste pro Cyclus trägt, die jeweils unabhängig voneinander ausgewählt sind aus der Gruppe C₁-C₄-Alkyl, Cyano, COO-(C₁-C₄-Alkyl), O-(C₁-C₄-Alkyl), N(C₁-C₄-Alkyl)₂, NH-(C₁-C₄-Alkyl), Fluor, Chlor, Brom oder C₁-C₄-Fluoralkyl wie zum Beispiel Trifluormethyl, 2,2,2-Trifluorethyl oder Pentafluorethyl.

Ganz besonders bevorzugt steht in Formel (I) Heteroaryl für 2-Thiophen-yl.

Bevorzugt steht W in Formel (I) für COOR¹, wobei R¹ für Wasserstoff oder C₁-C₈-Alkyl steht.

Bevorzugt steht in Formel (I) R¹ für C₁-C₁₂-Alkyl, Phenyl, o-, m-, p-Tolyl, p-Nitrophenyl oder Benzyl.

Besonders bevorzugt steht in Formel (I) R¹ für Methyl, Ethyl, 2-Ethoxyethyl, n-Propyl, Isopropyl, n-Butyl, tert.-Butyl, n-Pentyl, Cyclohexyl und n-Hexyl sowie für Trifluormethyl, Chlormethyl, Benzyl und Phenyl sowie für 1,5-Pentylen, 1,4-Butylen und 1,3-Propylen.

Ganz besonders bevorzugte Verbindungen der Formel (I) sind Methyl-3-oxo-3-(4-pyridinyl)propanoat, Ethyl-3-oxo-3-(4-pyridinyl)propanoat, Isopropyl-3-oxo-3-(4-pyridinyl)propanoat, Teit.-butyl-3-oxo-3-(4-pyridinyl)propanoat, 2-Ethylhexyl-3-oxo-3-(4-pyridinyl)propanoat, 3-oxo-3-(4-pyridinyl)propanamid, N,N-Dimethyl-3-oxo-3-(4-pyridinyl)propanamid, N-Methyl-3-oxo-3-(4-pyridinyl)propanamid, N,N-Diethyl-3-oxo-3-(4-pyridinyl)propanamid, N-Ethyl-3-oxo-3-(4-pyridinyl)propanamid, 3-Oxo-3-(N-piperidinyl)-1-(4-pyridinyl)-1-propanon, 3-Oxo-3-(N-pyrrolidinyl)-1-(4-pyridinyl)-1-propanon, 3-Oxo-3-(4-pyridinyl)propannitril, Methyl-3-oxo-3-(3-pyridinyl)propanoat, Ethyl-3-oxo-3-(3-pyridinyl)propanoat, Isopropyl-3-oxo-3-(3-pyridinyl)propanoat, Tert.-butyl-3-oxo-3-(3-pyridinyl)propanoat, 2-Ethylhexyl-3-oxo-3-(3-pyridinyl)propanoat, 3-Oxo-3-(3-pyridinyl)propanamid, N,N-Dimethyl-3-oxo-3-(3-pyridinyl)propanamid, N-Methyl-3-oxo-3-(3-pyridinyl)propanamid, N,N-Diethyl-3-oxo-3-(3-pyridinyl)propanamid, N-Ethyl-3-oxo-3-(3-pyridinyl)propanamid, 3-Oxo-3-(N-piperidinyl)-1-(3-pyridinyl)-1-propanon, 3-Oxo-3-(N-pyrrolidinyl)-1-(3-pyridinyl)-1-propanon, 3-Oxo-3-(3-pyridinyl)propannitril, Methyl-3-oxo-3-(2-pyridinyl)propanoat, Ethyl-3-oxo-3-(2-pyridinyl)propanoat, Isopropyl-3-oxo-3-(2-pyridinyl)propanoat, Tert.-butyl-3-oxo-3-(2-pyridinyl)propanoat, 2-Ethylhexyl-3-oxo-3-(2-pyridinyl)propanoat, 3-Oxo-3-(2-pyridinyl)propanamid, N,N-Dimethyl-3-oxo-3-(2-pyridinyl)propanamid, N-Methyl-3-oxo-3-(2-pyridinyl)propanamid, N,N-Diethyl-3-oxo-3-(2-pyridinyl)propanamid, N-Ethyl-3-oxo-3-(2-pyridinyl)propanamid, 3-Oxo-3-(N-piperidinyl)-1-(2-pyridinyl)-1-propanon, 3-Oxo-3-(N-pyrrolidinyl)-1-(2-pyridinyl)-1-propanon, 3-Oxo-3-(2-pyridinyl)propannitril, Methyl-3-oxo-3-(5-pyrimidinyl)propanoat, Ethyl-3-oxo-3-(5-pyrimidinyl)propanoat, Isopropyl-3-oxo-3-(5-pyrimidinyl)propanoat, Tert.-butyl-3-oxo-3-(5-pyrimidinyl)-propanoat, 2-Ethylhexyl-3-oxo-3-(5-pyrimidinyl)propanoat, 3-Oxo-3-(5-pyrimidinyl)propanamid, N,N-Dimethyl-3-oxo-3-(5-pyrimidinyl)propanamid, N-Methyl-3-oxo-3-(5-pyrimidinyl)propanamid, N,N-Diethyl-3-oxo-3-(5-pyrimidinyl)propanamid, N-Ethyl-3-oxo-3-(5-pyrimidinyl)propanamid, 3-Oxo-3-(N-piperidinyl)-1-(5-pyrimidinyl)-1-propanon, 3-Oxo-3-(N-pyrrolidinyl)-1-(5-pyrimidinyl)-1-propanon, 3-Oxo-3-(5-pyrimidinyl)propannitril, Methyl-3-oxo-3-(4-pyrimidinyl)propanoat, Ethyl-3-oxo-3-(4-pyrimidinyl)propanoat, Isopropyl-3-oxo-3-(4-pyrimidinyl)propanoat, Tert.-butyl-3-oxo-3-(4-pyrimidinyl)propanoat, 2-Ethylhexyl-3-oxo-3-(4-pyrimidinyl)propanoat, 3-Oxo-3-(4-pyrimidinyl)propanamid, N,N-Dimethyl-3-oxo-3-(4-pyrimidinyl)propanamid, N-Methyl-3-oxo-3-(4-pyrimidinyl)propanamid, N,N-Diethyl-3-oxo-3-(4-pyrimidinyl)propanamid, N-Ethyl-3-oxo-3-(4-pyrimidinyl)propanamid, 3-Oxo-3-(N-piperidinyl)-1-(4-pyrimidinyl)-1-propanon, 3-Oxo-3-(N-pyrrolidinyl)-1-(4-pyrimidinyl)-1-propanon, 3-Oxo-3-(4-pyrimidinyl)propannitril, Methyl-3-oxo-3-(2-pyrimidinyl)propanoat, Ethyl-3-oxo-3-(2-pyrimidinyl)propanoat, Isopropyl-3-oxo-3-(2-pyrimidinyl)propanoat, Tert.-butyl-3-oxo-3-(2-pyrimidinyl)propanoat, 2-Ethylhexyl-3-oxo-3-(2-pyrimidinyl)propanoat, 3-Oxo-3-(2-pyrimidinyl)propanamid, N,N-Dimethyl-3-oxo-3-(2-pyrimidinyl)propanamid, N-Methyl-3,-oxo-3-(2-pyrimidinyl)-propanamid, N,N-Diethyl-3-oxo-3-(2-pyrimidinyl)propanamid, N-Ethyl-3-oxo-3-(2-pyrimidinyl)propanamid, 3-Oxo-3-(N-piperidinyl)-1-(2-pyrimidinyl)-1-propanon, 3-Oxo-3-(N-pyrrolidinyl)-1-(2-pyrimidinyl)-1-propanon, 3-Oxo-3-(2-pyrimidinyl)-propannitril, Ethyl-3-(6-chloro-3-pyridinyl)-3-oxopropanoat, Ethyl-3-(2,6-dichloro-3-pyridinyl)-3-oxopropanoat, Ethyl-3-oxo-3-(4,5,6-trichloro-3-pyridinyl)propanoat, Ethyl-3-(2,6-dichloro-5-fluoro-3-pyridinyl)-3-oxopropanoat, Methyl-3-(3-chloro-1-benzothien-2-yl)-3-oxopropanoat, Methyl-3-oxo-3-(3-thiophen-yl)propanoat, Ethyl-3-oxo-3-(3-thiophen-yl)propanoat, Isopropyl-3-oxo-3-(3-thiophen-yl)propanoat, Tert.-butyl-3-oxo-3-(3-thiophen-yl)propanoat, 2-Ethylhexyl-3-oxo-3-(3-thiophen-yl)-propanoat, 3-Oxo-3-(3-thiophen-yl)propanamid, N,N-Dimethyl-3-oxo-3-(3-thiophenyl)propanamid, N-Methyl-3-oxo-3-(3-thiophen-yl)propanamid, N,N-Diethyl-3-oxo-3-(3-thiophen-yl)propanamid, N-Ethyl-3-oxo-3-(3-thiophen-yl)propanamid, 3-Oxo-3-(N-piperidinyl)-1-(3-thiophen-yl)-1-propanon, 3-Oxo-3-(N-pyrrolidinyl)-1-(3-thiophen-yl)-1-propanon, 3-Oxo-3-(3-thiophen-yl)propannitril, Methyl-3-oxo-3-(2-thiophen-yl)propanoat, Ethyl-3-oxo-3-(2-thiophen-yl)propanoat, Isopropyl-3-oxo-3-(2-thiophen-yl)propanoat, Tert.-butyl-3-oxo-3-(2-thiophen-yl)propanoat, 2-Ethylhexyl-3-oxo-3-(2-thiophen-yl)propanoat, 3-Oxo-3-(2-thiophen-yl)propanamid, N,N-Dimethyl-3-oxo-3-(2-thiophen-yl)propanamid, N,N-Diethyl-3-oxo-3-(2-thiophen-yl)-propanamid, N-Ethyl-3-oxo-3-(2-thiophen-yl)propanamid, 3-Oxo-3-(N-piperidinyl)-1-(2-thiophen-yl)-1-propanon, 3-Oxo-3-(N-pyrrolidinyl)-1-(2-thiophen-yl)-1-propanon, 3-Oxo-3-(2-thiophen-yl)propannitril, Methyl-3-oxo-3-(3-pyrrolyl)propanoat, Ethyl-3-oxo-3-(3-pyrrolyl)propanoat, Isopropyl-3-oxo-3-(3-pyrrolyl)propanoat, Tert.-butyl-3-oxo-3-(3-pyrrolyl)propanoat, 2-Ethylhexyl-3-oxo-3-(3-pyrrolyl)propanoat, 3-Oxo-3-(3-pyrrolyl)propanamid, N,N-Dimethyl-3-oxo-3-(3-pyrrolyl)propanamid, N-Methyl-3-oxo-3-(3-pyrrolyl)propanamid, N,N-Diethyl-3-oxo-3-(3-pyrrolyl)propanamid, N-Ethyl-3-oxo-3-(3-pyrrolyl)propanamid, 3-Oxo-3-(N-piperidinyl)-1-(3-pyrrolyl)-1-propanon, 3-Oxo-3-(N-pyrrolidinyl)-1-(3-pyrrolyl)-1-propanon, 3-Oxo-3-(3-pyrrolyl)propannitril, Methyl-3-oxo-3-(2-pyrrolyl)propanoat, Ethyl-3-oxo-3-(2-pyrrolyl)propanoat, Isopropyl-3-oxo-3-(2-pyrrolyl)propanoat, Tert.-butyl-3-oxo-3-(2,-pyrrolyl)propanoat, 2-Ethylhexyl-3-oxo-3-(2-pyrrolyl)propanoat, 3-Oxo-3-(2-pyrrolyl)propanamid, N,N-Dimeihyl-3-oxo-3-(2-pyrrolyl)propanamid, N-Methyl-3-oxo-3-(2-pyrrolyl)propanamid, N,N-Diethyl-3-oxo-3-(2-pyrrolyl)propamamid, N-Ethyl-3-oxo-3-(2-pyrrolyl)propanamid, 3-Oxo-3-(N-piperidinyl)-1-(2-pyrrolyl)-1-propanon, 3-Oxo-3-(N-pyirolidinyl)-1-(2-pyrrolyl)-1-propanon, 3-Oxo-3-(2-pyrrolyl)propannitril, Methyl-3-oxo-3-(1-thiazolyl)propanoat, Ethyl-3-oxo-3-(1-thiazolyl)propanoat, Isopropyl-3-oxo-3-(1-thiazolyl)propanoat, Tert.-butyl-3-oxo-3-(1-thiazolyl)propanoat, 2-Ethylhexyl-3-oxo-3-(1-thiazolyl)propanoat, 3-Oxo-3-(1-thiazolyl)propanamid, N,N-Dimethyl-3-oxo-3-(1-thiazolyl)-propanamid, N-Methyl-3-oxo-3-(1-thiazolyl)propanamid, N,N-Diethyl-3-oxo-3-(1-thiazolyl)propanamid, N-Ethyl-3-oxo-3-(1-thiazolyl)propanamid, 3-Oxo-3-(N-piperidinyl)-1-(1-thiazolyl)-1-propanon, 3-Oxo-3-(N-pyrrolidinyl)-1-(1-thiazolyl)-1-propanon, 3-Oxo-3-(1-thiazolyl)propannitril, Methyl-3-oxo-3-(4-thiazolyl)propanoat, Ethyl-3-oxo-3-(1-thiazolyl)proparoat, Isopropyl-3-oxo-3-(1-thiazolyl)propanoat, Tert.-butyl-3-oxo-3-(1-thiazolyl)propanoat, 2-Ethylhexyl-3-oxo-3-(1-thiazolyl)propanoat, 3-Oxo-3-(1-thiazolyl)propanamid, N,N-Dimethyl-3-oxo-3-(1-thiazolyl)propanamid, N-Methyl-3-oxo-3-(1-thiazolyl)propanamid, N,N-Diethyl-3-oxo-3-(1-thiazolyl)propanamid, N-Ethyl-3-oxo-3-(1-thiazolyl)propanamid, 3-Oxo-3-(N-piperidinyl)-1-(1-thiazolyl)-1-propanon, 3-Oxo-3-(N-pyrrolidinyl)-1-(1-thiazolyl)-1-propanon, 3-Oxo-3-(1-thiazolyl)propannitril, Methyl-3-oxo-3-(1-oxazolyl)propanoat, Ethyl-3-oxo-3-(1-thiazolyl)propanoat, Isopropyl-3-oxo-3-(1-thiazolyl)propanoat, Tert.-butyl-3-oxo-3-(1-thiazolyl)propanoat, 2-Ethylhexyl-3-oxo-3-(1-thiazolyl)propanoat, 3-Oxo-3-(1-thiazolyl)propanamid, N,N-Dimethyl-3-oxo-3-(1-thiazolyl)propanamid, N-Methyl-3-oxo-3-(1-thiazolyl)propanamid, N,N-Diethyl-3-oxo-3-(1-thiazolyl)propanamid, N-Ethyl-3-oxo-3-(1-thiazolyl)propanamid, 3-Oxo-3-(N-piperidinyl)-1-(1-thiazolyl)-1-propanon, 3-Oxo-3-(N-pyrrolidinyl)-1-(1-thiazolyl)-1-propanon, 3-Oxo-3-(1-thiazolyl)propannitril, Methyl-3-oxo-3-(1-oxazolyl)propanoat, Ethyl-3-oxo-3-(1-thiazolyl)propanoat, Isopropyl-3-oxo-3-(1-thiazolyl)propanoat, Tert.-butyl-3-oxo-3-(1-thiazolyl)propanoat, 2-Ethylhexyl-3-oxo-3-(1-thiazolyl)-propanoat, 3-Oxo-3-(1-thiazolyl)propanamid, N,N-Dimethyl-3-oxo-3-(1-thiazolyl)propanamid, N-Methyl-3-oxo-3-(1-thiazolyl)propanamid, N,N-Diethyl-3-oxo-3-(1-thiazolyl)propanamid, N-Ethyl-3-oxo-3-(1-thiazolyl)propanamid, 3-Oxo-3-(N-piperidinyl)-1-(1-thiazolyl)-1-propanon, 3-Oxo-3-(N-pyrrolidinyl)-1-(1-thiazolyl)-1-propanon, 3-Oxo-3-(1-thiazolyl)propannitril, Methyl-3-oxo-3-(2-oxazolyl)propanoat, Ethyl-3-oxo-3-(1-thiazolyl)propanoat, Isopropyl-3-oxo-3-(1-thiazolyl)propanoat, Tert.-butyl-3-oxo-3-(1-thiazolyl)propanoat, 2-Ethylhexyl-3-oxo-3-(1-thiazolyl)propanoat, 3-Oxo-3-(1-thiazolyl)propanamid, N,N-Dimethyl-3-oxo-3-(1-thiazolyl)propanamid, N-Methyl-3-oxo-3-(1-thiazolyl)propanamid, N,N-Diethyl-3-oxo-3-(1-thiazolyl)propanamid, N-Ethyl-3-oxo-3-(1-thiazolyl)propanamid, 3-Oxo-3-(N-piperidinyl)-1-(1-thiazolyl)-1-propanon, 3-Oxo-3-(N-pyrrolidinyl)-1-(1-thiazolyl)-1-propanon, 3-Oxo-3-(1-thiazolyl)propannitril, Methyl-3-oxo-3-(4-oxazolyl)propanoat, Ethyl-3-oxo-3-(4-oxazolyl)propanoat, Isopropyl-3-oxo-3-(4-oxazolyl)propanoat, Tert.-butyl-3-oxo-3-(4-oxazolyl)propanoat, 2-Ethylhexyl-3-oxo-3-(4-oxazolyl)-propanoat, 3-Oxo-3-(4-oxazolyl)propanamid, N,N-Dimethyl-3-oxo-3-(4-oxazolyl)propanamid, N-Methyl-3-oxo-3-(4-oxazolyl)propanamid, N,N-Diethyl-3-oxo-3-(4-oxazolyl)propanamid, N-Ethyl-3-oxo-3-(4-oxazolyl)propanamid, 3-Oxo-3-(N-piperidinyl)-1-(4-oxazolyl)-1-propanon, 3-Oxo-3-(N-pyrrolidinyl)-1-(4-oxazolyl)-1-propanon, 3-Oxo-3-(4-oxazolyl)propannitril, Methyl-3-oxo-3-(3-pyrazolyl)propanoat, Ethyl-3-oxo-3-(3-pyrazolyl)propanoat, Isopropyl-3-oxo-3-(3-pyrazolyl)-propanoat, Tert.-butyl-3-oxo-3-(3-pyrazolyl)propanoat, 2-Ethylhexyl-3-oxo-3-(3-pyrazolyl)propanoat, 3-Oxo-3-(3-pyrazolyl)propanamid, N,N-Dimethyl-3-oxo-3-(3-pyrazolyl)propanamid, N-Methyl-3-oxo-3-(3-pyrazolyl)propanamid, N,N-Diethyl-3-oxo-3-(3-pyrazolyl)propanamid, N-Ethyl-3-oxo-3-(3-pyrazolyl)propanamid, 3-Oxo-3-(N-piperidinyl)-1-(3-pyrazolyl)-1-propanon, 3-Oxo-3-(N-pyrrolidinyl)-1-(3-pyrazolyl)-1-propanon, 3-Oxo-3-(3-pyrazolyl)propannitril, Methyl-3-oxo-3-(4-pyrazolyl)-propanoat, Ethyl-3-oxo-3-(4-pyrazolyl)propanoat, Isopropyl-3-oxo-3-(4-pyrazolyl)-propanoat, Tert.-butyl-3-oxo-3-(4-pyrazolyl)propanoat, 2-Ethylhexyl-3-oxo-3-(4-pyrazolyl)propanoat, 3-Oxo-3-(4-pytazolyl)propanamid, N,N-Dimethyl-3-oxo-3-(4-pyrazolyl)propanamid, N-Methyl-3-oxo-3-(4-pyrazolyl)propanamid, N,N-Diethyl-3-oxo-3-(4-pyrazolyl)propanamid, N-Ethyl-3-oxo-3-(4-pyrazolyl)propanamid, 3-Oxo-3-(1-piperidiriyl)-1-(4-pyrazolyl)-1-proparion, 3-Oxo-3-(N-pyrrolidinyl)-1-(4-pyrazolyl)-1-propanon, 3-Oxo-3-(4-pyrazolyl)propannitril, Methyl-3-oxo-3-(2-imidazolyl)propanoat, Ethyl-3-oxo-3-(2-imidazolyl)propanoat, Isopropyl-3-oxo-3-(2-imidazolyl)propanoat, Tert.-butyl-3-oxo-3-(2-imidazolyl)propanoat, 2-Ethylhexyl-3-oxo-3-(2-imidazolyl)propanoat, 3-Oxo-3-(2-imidazolyl)propanamid, N,N-Dimethyl-3-oxo-3-(2-imidazolyl)propanamid, N-Methyl-3-oxo-3-(2-imidazolyl)propanamid, N,N-Diethyl-3-oxo-3-(2-imidazolyl)propanamid, N-Ethyl-3-oxo-3-(2-imidazolyl)-propanamid, 3-Oxo-3-(N-piperidinyl)-1-(2-imidazolyl)-1-propanon, 3-Oxo-3-(N-pyrrolidinyl)-1-(2-imidazolyl)-1-propanon, 3-Oxo-3-(2-imidazolyl)propannitril, Methyl-3-oxo-3-(4-imidazolyl)propanoat, Ethyl-3-oxo-3-(4-imidazolyl)propanoat, Isopropyl-3-oxo-3-(4-imidazolyl)propanoat, Tert.-butyl-3-oxo-3-(4-imidazolyl)propanoat, 2-Ethylhexyl-3-oxo-3-(4-imidazolyl)propanoat, 3-Oxo-3-(4-imidazolyl)propanamid, N,N-dimethyl-3-oxo-3-(4-imidazolyl)propanamid, N-Methyl-3-oxo-3-(4-imidazolyl)propanamid, N,N-Diethyl-3-oxo-3-(4-imidazolyl)propanamid, N-Ethyl-3-oxo-3-(4-imidazolyl)propanamid, 3-Oxo-3-(N-piperidinyl)-1-(4-imidazolyl)-1-propanon, 3-Oxo-3-(N-pyrrolidinyl)-1-(4-imidazolyl)-1-propanon, 3-Oxo-3-(4-imidazolyl)-propannitril, Methyl-3-oxo-3-(5-imidazolyl)propanoat, Ethyl-3-oxo-3-(5-imidazolyl)propanoat, Isopropyl-3-oxo-3-(5-imidazolyl)propanoat, Tert.-butyl-3-oxo-3-(5-imidazolyl)propanoat, 2-Ethylhexyl-3-oxo-3-(5-imidazolyl)propanoat, 3-Oxo-3-(5-imidazolyl)propanamid, N,N-Dimethyl-3-oxo-3-(5-imidazolyl)propanamid, N-Methyl-3-oxo-3-(5-imidazolyl)propanamid, N,N-Diethyl-3-oxo-3-(5-imidazolyl)-propanamid, N-Ethyl-3-oxo-3-(5-imidazolyl)propanamid, 3-Oxo-3-(N-piperidinyl)-1-(5-imidazolyl)-1-propanon, 3-Oxo-3-(N-pyrrolidinyl)-1-(5-imidazolyl)-1-propanon, 3-Oxo-3-(5-imidazolyl)propannitril, Methyl-3-oxo-3-(3-furanyl)propanoat, Ethyl-3-oxo-3-(3-furanyl)propanoat, Isopiopyl-3-oxo-3-(3-furanyl)propanoat, Tert.-bütyl-3-oxo-3-(3-furanyl)propanoat, 2-Ethylhexyl-3-oxo-3-(3-furanyl)propanoat, 3-Oxo-3-(3-furanyl)propanamid, N,N-Dimethyl-3-oxo-3-(3-furanyl)propanamid, N-Methyl-3-oxo-3-(3-kranyl)propanamid, N,N-Diethyl-3-oxo-3-(3-furanyl)propanamid, N-Ethyl-3-oxo-3-(3-furanyl)propanamid, 3-Oxo-3-(N-piperidinyl)-1-(3-furanyl)-1-propanon, 3-Oxo-3-(N-pyrrolidinyl)-1-(3-furanyl)-1-propanon, 3-Oxo-3-(3-furanyl)propannitril, Methyl-3-oxo-3-(2-furanyl)propanoat, Ethyl-3-oxo-3-(2-Kranyl)propanoat, Isopropyl-3-oxo-3-(2-furanyl)propanoat, Tert.-butyl-3-oxo-3-(2-furanyl)propanoat, 2-Ethylhexyl-3-oxo-3-(2-furanyl)proparioat, 3-Oxo-3-(2-furanyl)-propanamid, N,N-Dimethyl-3-oxo-3-(2-furanyl)propanamid, N-Methyt-3-oxo-3-(2-furanyl)propariamid, N,N-Diethyl-3-oxo-3-(2-furanyl)propanamid, N-Ethyl-3-oxo-3-(2-furanyl)propanamid, 3-Oxo-3-(N-piperidinyl)-1-(2-furanyl)-1-propanon, 3-Oxo-3-(N-pyrrolidinyl)-1-(2-furanyl)-1-propanon, 3-Oxo-3-(2-furanyl)propannitril, Methyl-3-oxo-3-(3-indolyl)propanoat, Ethyl-3-oxo-3-(3-indolyl)propanoat, Isopropyl-3-oxo-3-(3-indolyl)propauoat, Tert.-butyl-3-oxo-3-(3-indolyl)propanoat, 2-Ethylhexyl-3-oxo-3-(3-indolyl)propanoat, 3-Oxo-3-(3-indolyl)propanamid, N,N-Dimethyl-3-oxo-3-(3-indolyl)propanamid, N-Methyl-3-oxo-3-(3-indolyl)propanamid, N,N-Diethyl-3-oxo-3-(3-indolyl)propanamid, N-Ethyl-3-oxo-3-(3-indolyl)propanamid, 3-Oxo-3-(N-piperidinyl)-1-(3-indolyl)-1-propanon, 3-Oxo-3-(N-pyrrolidinyl)-1-(3-indolyl)-1-propanon, 3-Oxo-3-(3-indolyl)propannitril, Methyl-3-oxo-3-(2-indolyl)propanoat, Ethyl-3-oxo-3-(2-indolyl)propanoat, Isopropyl-3-oxo-3-(2-indolyl)propanoat, Tert.-butyl-3-oxo-3-(2-indolyl)propanoat, 2-Ethylhexyl-3-oxo-3-(2-indolyl)propanoat, 3-Oxo-3-(2-indolyl)propanamid, N,N-Dimethyl-3-oxo-3-(2-indolyl)propanamid, N-Methyl-3-oxo-3-(2-indolyl)propanamid, N,N-Diethyl-3-oxo-3-(2-indolyl)propanamid, N-Ethyl-3-oxo-3-(2-indolyl)propanarnid, 3-Oxo-3-(N-piperidinyl)-1-(2-indolyl)-1-propanon, 3-Oxo-3-(N-pyrrolidinyl)-1-(2-indolyl)-1-propanon, 3-Oxo-3-(2-indolyl)propannitril, Methyl-3-oxo-3-(3-benzofuranyl)propanoat, Ethyl-3-oxo-3-(3-benzofuranyl)propanoat, Isopropyl-3-oko-3-(3-benzofuranyl)propanoat, Tert.-butyl-3-oxo-3-(3-benzofuranyl)propanoat, 2-Ethylhexyl-3-oxo-3-(3-benzofuranyl)propanoat, 3-Oxo-3-(3-benzofuranyl)propanamid, N,N-Dimethyl-3-oxo-3-(3-benzofuranyl)propanamid, N-Methyl-3-oxo-3-(3-benzofuranyl)propanahid, N,N-Diethyl-3-oxo-3-(3-benzofuranyl)propanamid, N-Ethyl-3-oxo-3-(3-benzofuranyl)propanamid, 3-Oxo-3-(N-piperidinyl)-1-(3-benzofuranyl)-1-propanon, 3-Oxo-3-(N-pyrrolidinyl)-1-(3-benzofuranyl)-1-propanon, 3-Oxo-3-(3-benzofuranyl)propannitril, Methyl-3-oxo-3-(2-benzofuranyl)propanoat, Ethyl-3-oxo-3-(2-benzofuranyl)propanoat, Isopropyl-3-oxo-3-(2-benzofuranyl)propanoat, Tert.-butyl-3-oxo-3-(2-benzofuranyl)propanoat, 2-Ethylhexyl-3-oxo-3-(2-benzofuranyl)propanoat, 3-Oxo-3-(2-benzofuranyl)propanamid, N,N-Dimethyl-3-oxo-3-(2-benzofuranyl)propanamid, N-Methyl-3-oxo-3-(2-benzofuranyl)propanamid, N,N-Diethyl-3-oxo-3-(2-benzofuranyl)propanamid, N-Ethyl-3-oxo-3-(2-benzofuranyl)propanamid, 3-Oxo-3-(N-piperidinyl)-1-(2-benzofuranyl)-1-propanon, 3-Oxo-3-(N-pyrrolidinyl)-1-(2-benzofuranyl)-1-propanon; 3-Oxo-3-(2-benzofuranyl)propannitril, Methyl-3-(2-benzothiophen-yl)-3-oxopropanoat, Ethyl-3-oxo-3-(2-benzothiophen-yl)propanoat, Isopropyl-3-oxo-3-(2-benzothiophen-yl)propanoat, Tert.-butyl-3-oxo-3-(2-benzothiophen-yl)propanoat, 2-Ethylhexyl-3-oxo-3-(2-benzothiophen-yl)propanoat, 3-Oxo-3-(2-benzothiophen-yl)propanamid, N,N-Dimethyl-3-oxo-3-(2-benzothiophenyl)propanamid, N-Methyl-3-oxo-3-(2-benzothiophen-yl)propanamid, N,N-Diethyl-3-oxo-3-(2-benzothiophen-yl)propanamid, N-Ethyl-3-oxo-3-(2-benzothiophen-yl)propanamid, 3-Oxo-3-(N-piperidinyl)-1-(2-benzothiophen-yl)-1-propanon, 3-Oxo-3-(N-pyrrolidinyl)-1-(2-benzothiophen-yl)-1-propanon, 3-Oxo-3-(2-benzothiophen-yl)propannitril, Methyl-3-(3-benzothiophenyl)-3-oxopropanoat, Ethyl-3-oxo-3-(3-benzothiophen-yl)propanoat, Isopropyl-3-oxo-3-(3-benzothiophen-yl)propanoat, Tert.-butyl-3-oxo-3-(3-benzothiophen-yl)-propanoat, 2-Ethylhexyl-3-oxo-3-(3-benzothiophen-yl)propanoat, 3-Oxo-3-(3-benzothiophen-yl)propanamid, N,N-Dimethyl-3-oxo-3-(3-benzothiophen-yl)propanamid, N-Methyl-3-oxo-3-(3-benzothiophen-yl)propanamid, N,N-Diethyl-3-oxo-3-(3-berizothiophen-yl)propanamid, N-Ethyl-3-oxo-3-(3-benzothiophen-yl)propanamid, 3-Oxo-3-(N-piperidmyl)-1-(3-benzothiophen-yl)-1-propanon, 3-Oxo-3-(N-pyrrolidinyl)-1-(3-benzothiophen-yl)-1-propanon, 3-Oxo-3-(3-benzothiophen-yl)propannitril, Methyl-3-(2-chinolinyl)-3-oxopropanoat, Ethyl-3-oxo-3-(2-chinolinyl)propanoat, Isopropyl-3-oxo-3-(2-chinolinyl)propanoat, Tert.-butyl-3-oxo-3-(2-chinolinyl)propanoat, 2-Ethylhexyl-3-oxo-3-(2-chinolinyl)propanoat, 3-Oxo-3-(2-chinolinyl)propanamid, N,N-Dimethyl-3-oxo-3-(2-chinolinyl)propanamid, N-Methyl-3-oxo-3-(2-chinolinyl)propanamid, N,N-Diethyl-3-oxo-3-(2-chinolinyl)-propanamid, N-Ethyl-3-oxo-3-(2-chinolinyl)propanamid, 3-Oxo-3-(N-piperidinyl)-1-(2-chinolinyl)-1-propanon, 3-Oxo-3-(N-pyrrolidinyl)-1-(2-chinolinyl)-1-propanon, 3-Oxo-3-(2-chinolinyl)propannitril, Methyl-3-(3-chinolinyl)-3-oxopropanoat, Ethyl-3-oxo-3-(3-chinolinyl)propanoat, Isopropyl-3-oxo-3-(3-chinolinyl)propanoat, Tert.-butyl-3-oxo-3-(3-chinolinyl)propanoat, 2-Ethylhexyl-3-oxo-3-(3-chinolinyl)propanoat, 3-Oxo-3-(3-chindlinyl)propanamid, N,N-Dimethyl-3-oxo-3-(3-chinolinyl)-propanamid, N-Methyl-3-oxo-3-(3-chinolinyl)propanamid, N,N-Diethyl-3-oxo-3-(3-chinolinyl)propanamid, N-Ethyl-3-oxo-3-(3-chinolinyl)propanamid, 3-Oxo-3-(N-piperidinyl)-1-(3-chinolinyl)-1-propanon, 3-Oxo-3-(N-pyn-olidmyl)-1-(3-chinolinyl)-1-propanon, 3-Oxo-3-(3-chinolinyl)propannitril, Methyl-3-(3-isochinolinyl)-3-oxopropanoat, Ethyl-3-oxo-3-(3-isochinolinyl)propanoat, Isopropyl-3-oxo-3-(3-isochinolinyl)propanoat, Tert.-butyl-3-oxo-3-(3-isochimolinyl)propanoat, 2-Ethylhexyl-3-oxo-3-(3-isochinolinyl)propanoat, 3-Oxo-3-(3-isochinolinyl)propanamid, N,N-Dimethyl-3-oxo-3-(3-isochinolinyl)propanamid, N-Methyl-3-oxo-3-(3-isochinolinyl)propanamid, N,N-Diethyl-3-oxo-3-(3-isochinolinyl)propanamid, N-Ethyl-3-oxo-3-(3-isochinolinyl)propanamid, 3-Oxo-3-(N-piperidinyl)-1-(3-isochinolinyl)-1-propanon, 3-Oxo-3-(N-pyrrolidinyl)-1-(3-isochinolinyl)-1-propanon, 3-Oxo-3-(3-isochinolinyl)propannitril, wobei Methyl-3-oxo-3-(2-thiophen-yl)propanoat und Ethyl-3-oxo-3-(2-thiophen-yl)propanoat noch weiter bevorzugt sind.

Das erfindungsgemäße Verfahren wird in Gegenwart eines Ruthenium enthaltenden Katalysators durchgeführt.

Beispielsweise und bevorzugt werden solche Katalysatoren verwendet; die Rutheniumkomplexe enthalten. Bevorzugte Rutheniumkomplexe sind solche, die durch Umsetzung von Verbindungen der Formel (II) mit Verbindungen der Formel (III) erhältlich sind, oder Komplexe der Formel (IV). Besonders bevorzugt werden solche Rutheniumkomplexe eingesetzt, die durch Umsetzung von Verbindungen der Formel (II) mit Verbindungen der Formel (III) erhältlich sind. In einer bevorzugten Ausführungsform beträgt dabei das molare Verhältnis von Verbindungen der Formel (III) und Verbindungen der Formel (II) 2:1 bis 3:1, besonders bevorzugt 2,01: bis 2,4:1.

Vorteilhafterweise werden Verbindungen der Formel (III) und Verbindungen der Formel (II) gemischt und die Mischung in organischem Lösungsmittel aufgenommen. Die resultierende Mischung kann weiterhin vorteilhafterweise vor Zugabe zur Reaktionsmischung mit einer Base, bevorzugt einem tertiären Amin, versetzt und beispielsweise und bevorzugt 10 bis 30 min gerührt werden, wobei die molare Menge tertiären Amins beispielsweise und bevorzugt 1:1 bis 3:1, besonders bevorzugt 1:1 bis 2:1 bezogen auf Verbindungen der Formel (III) beträgt.

Für organische Lösungsmittel und tertiäre Amine gelten die ,gleichen Angaben und Vorzugsbereiche, die weiter unten detailliert beschrieben werden.

In den Verbindungen der Formel (II)

[RuX₂(Aren)]₂ (II)

steht
- Aren: für eine koordinierte aromatische Verbindung mit 6 bis 12 Ringkohlenstoffatomen, die weiterhin mit bis zu 6 Resten substituiert sein kann, die jeweils unabhängig voneinander ausgewählt sind aus der Gruppe C₁-C₈-Alkyl, Benzyl und Phenyl und
- X: beispielsweise und bevorzugt für Chlor, Brom oder Iod, besonders bevorzugt für Chlor.

Bevorzugt steht Aren für Benzol oder Naphthalin, das mit bis zu 6 Resten substituiert sein kann, die jeweils unabhängig voneinander ausgewählt sind aus der Gruppe Methyl, Ethyl,n-Propyl, Isopropyl und tert.-Butyl.

Bevorzugt steht Aren für Mesitylen, Cumol oder Benzol.

Besonders bevorzugte Verbindungen der Formel (II) sind Benzoldichlororuthenium-Dimer, Mesitylendichlororuthenium-Dimer und Cumoldichlororuthenium-Dimer, wobei Cumoldichlororuthenium-Dimer noch weiter bevorzugt ist.

In der Formel (III) 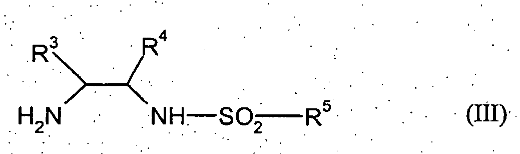 stehen.
- R³ und R: jeweils unabhängig voneinander beispielsweise für C₁-C₂₀-Alkyl, C₄-C₁₅-Aryl oder C₅-C₁₆-Arylalkyl oder R und R⁴ zusammen für einen geradkettigen oder verzweigten C₃-C₁₂-Alkylen-Rest, und
- R⁵: für C₁-C₂₀-Alkyl, C₁-C₂₀-Fluoralkyl oder C₄-C₁₅-Aryl.

Bevorzugt stehen R³ und R⁴ jeweils identisch für Phenyl oder zusammen für geradkettiges C₃-C₈-Alkylen wie zum Beispiel 1,3-Pentylen oder 1,4-Butylen, besonders bevorzugt stehen R³ und R⁴ jeweils identisch für Phenyl.
- R⁵: steht bevorzugt für C₁-C₄-Alkyl, C₁-C₄-Fluoralkyl, Phenyl oder Naphthyl, das mit keinem, einem, zwei, drei, vier oder fünf Resten weiter substituiert sein kann, die ausgewählt sind aus der Gruppe C₁-C₄-Alkyl, C₁-C₄-Alkoxy, C₁-C₄-Fluoralkyl, Fluor und Chlor.
- R⁵: steht besonders bevorzugt für Methyl, Trifluormethyl, Pentafluorethyl, Nonafluorbutyl, Phenyl, p-Tolyl, p-Ethylphenyl, p-Anisyl, p-Ethoxyphenyl, p-Chlorphenyl, 2,4,6-Trimethylphenyl, 2,4,6-Triisopropylphenyl, p-Fluorphenyl, Pentafluorphenyl, und Naphthyl.
- R⁵: steht ganz besonders bevorzugt für p-Tolyl, Phenyl, Naphtyl.
- R⁵: steht noch weiter bevorzugt für p-Tolyl.

Bevorzugt weisen die Verbindungen der Formel (III) eine Stereoisomerenreinheit von 90 % oder mehr, besonders bevorzugt von 95 % oder mehr und ganz besonders bevorzugt von 98,5 % oder mehr auf.

Als Verbindungen der Formel (III) seien genannt:
N-[(1R,2R) und (1S,2S)-2-amino-1,2-diphenylethyl]-p-tölylsulfonamid, N-[(1R,2R) und (1S,2S)-2-amino-1,2-diphenylethyl]-o-tolylsulfonamid, N-[(1R,2R) und (1S,2S)-2-amino-1,2-diphenylethyl]-m-tolylsulfonamid, N-[(1R,2R) und (1S,2S)-2-amino-1,2-diphenylethyl]-phenylsulfonamid, N-[(1R,2R) und (1S,2S)-2-amino-1,2-diphenylethyl]-4-ethylphenylsulfonamid, N-[(1R,2R) und (1S,2S)-2-amino-1,2-diphenylethyl]-3-ethylphenylsulfonamid, N-[(1R,2R) und (1S,2S)-2-amino-1,2-diphenylethyl]-2-ethylphenylsulfonamid, N-[(1R,2R) und (1S,2S)-2-amino-1,2-diphenylethyl]-2,4,6-trimethylphenylsulfonamid, N-[(1R,2R) und (1S,2S)-2-amino-1,2-diphenylethyl]-2,4,6-triisopropylphenylsulfonamid, N-[(1R,2R) und (1S,2S)-2-amino-1,2-diphenylethyl]-4-chlorphenylsulfonamid, N-[(1R,2R) und (1S,2S)-2-anino-1,2-diphenylethyl]-3-chlorphenylsulfonamid, N-[(1R,2R) und (1S,2S)-2-anino-1,2-diphenylethyl]-2-chlorphenylsulfonamid, N-[(1R,2R) und (1S,2S)-2-amino-1,2-diphenylethyl]-4-fluorphenylsulfonamid, N-[(1R,2R) und (1S,2S)-2-amino-1,2-diphenylethyl]-3-fluorphenylsulfonamid, N-[(1R,2R) und (1S,2S)-2-amino-1,2-diphenylethyl]-2-fluorphenylsulfonamid, N-[(1R,2R) und (1S,2S)-2-amino-1,2-diphenylethyl]-4-methoxyphenylsulfonamid, N-[(1R,2R) und (1S,2S)-2-amino-1,2-diphenylethyl]-3-methoxyphenylsulfonamid, N-[(1R,2R) und (1S,2S)-2-amino-1,2-diphenylethyl]-2-methoxyphenylsulfonamid, N-[(1R,2R) und (1S,2S)-2-amino-1,2-diphenylethyl]-1-naphtylsulfonamid, N-[(1R,2R) und (1S,2S)-2-amino-1,2-diphenylethyl]-2-naphtylsulfonamid, N-[(1R,2R) und (1S,2S)-2-amino-1,2-diphenylethyl]-pentafluorphenylsulfonamid, N-[(1R,2R) und (1S,2S)-2-amino-1,2-diphenylethyl]-methansulfonamid, N-[(1R,2R) und (1S,2S)-2-amino-1,2-diphenylethyl]-trifluormethansulfonamid, N-[(1R,2R) und (1S,2S)-2-aminocyclohexyl]-p-tolylsulfonamid, N-[(1R,2R) und (1S,2S)-2-aminocyclohexyl]-o-tolylsulfonamid, N-[(1R,2R) und (1S,2S)-2-aminocyclohexyl]-m-tolylsulfonamid, N-[(1R,2R) und (1S,2S)-2-aminocyclohexyl]-phenylsulfonamid, N-[(1R,2R) und (1S,2S)-2-aminocyclohexyl]-4-ethylphenylsulfonamid, N-[(1R,2R) und (1S,2S)-2-aminocyclohexyl]-3-ethylphenylsulfonamid, N-[(1R,2R) und (1S,2S)-2-aminocyclohexyl]-2-ethylphenylsulfonamid, N-[(1R,2R) und (1S,2S)-2-aminocyclohexyl]-2,4,6-trimethylphenylsulfonamid, N-[(1R,2R) und (1S,2S)-2-aminocyclohexyl]-2,4,6-triisopropylphenylsulfonamid, N-[(1R,2R) und (1S,2S)-2-aminocyclohexyl]-4-chlorphenylsulfonamid, N-[(1R,2R) und (1S,2S)-2-aminocyclohexyl]-3-chlorphenylsulfonamid, N-[(1R,2R) und (1S,2S)-2-aminocyclohexyl]-2-chlorphenylsulfonamid, N-[(1R,2R) und (1S,2S)-2-aminocyclohexyl]-4-fluorphenylsulfonamid, N-[(1R,2R) und (1S,2S)-2-aminoclohexyl]-3-fluorphenylsulfonamid, N-[(1R,2R) und (1S,2S)-2-aminocyclohexyl]-2-fluorphenylsulfonamid, N-[(1R,2R) und (1S,2S)-2-aminocyclohexyl]-4-methoxyphenylsulfonamid, N-[(1R,2R) und (1S,2S)-2-aminocyclohexyl]-3-methoxyphenylsulfonamid, N-[(1R,2R) und (1S,2S)-2-aminocyclohexyl]-2-methoxyphenylsulfonamid, N-[(1R,2R) und (1S,2S)-2-aminocyclohexyl]-1-naphtylsulfonamid, N-[(1R,2R) und (1S,2S)-2-aminocyclohexyl]-2-naphtylsulfonamid, N-[(1R,2R) und (1S,2S)-2-aminocyclohexyl]-pentafluorphenylsulfonamid, N-[(1R,2R) und (1S,2S)-2-aminocyclohexyl]-methansulfonamid, N-[(1R,2R) und (1S,2S)-2-aminocyclohexyl]-trifluormethansulfonamid, N-[(1R,2R) und (1S,2S)-2-aminocyclopentyl]-p-tolylsulfonamid, N-[(1R,2R) und (1S,2S)-2-aminocyclopentyl]-o-tolylsulfonamid, N-[(1R,2R) und (1S,2S)-2-aminocyclopentyl]-m-tolylsulfonamid, N-[(1R,2R) und (1S,2S)-2-aminocyclopentyl]-pheriylsulfonamid, N-[(1R,2R) und (1S,2S)-2-aminocyclopentyl]-4-ethylphenylsulfonamid, N-[(1R,2R) und (1S,2S)-2-aminocyclopentyl]-3-ethylphenylsulfonamid, N-[(1R,2R) und (1S,2S)-2-aminocyclopentyl]-2-ethylphenylsulfonamid, N-[(1R,2R) und (1S,2S)-2-aminocyclopentyl]-2,4,6-trimethylphenylsulfonamid, N-[(1R,2R) und (1S,2S)-2-aminocyclopentyl]-2,4,6-triisopropylphenylsulfonamid, N-[(1R,2R) und (1S,2S)-2-aminocyclopentyl]-4-chlorphenylsulfonamid, N-[(1R,2R) und (1S,2S)-2-aminocyclopentyl]-3-chlorphenylsulfonamid, N-[(1R,2R) und (1S,2S)-2-aminocyclopentyl]-2-chlorphenylsulfonamid, N-[(1R,2R) und (1S,2S)-2-aminocyclopentyl]-4-fluorphenylsulfonamid, N-[(1R,2R) und (1S,2S)-2-aminocyclopentyl]-3-fluorphenylsulfonamid, N-[(1R,2R) und (1S,2S)-2-aminocyclopentyl]-2-fluorphenylsulfonamid, N-[(1R,2R) und (1S,2S)-2-aminocyclopentyl]-4-methoxyphenylsulfonamid, N-[(1R,2R) und (1S,2S)-2-aminocyclopentyl]-3-methoxyphenylsulfonamid, N-[(1R,2R) und (1S,2S)-2-aminocyclopentyl]-2-methoxyphenylsulfonamid, N-[(1R,2R) und (1S,2S)-2-aminocyclopentyl]-1-naphtylsulfonamid, N-[(1R,2R) und (1S,2S)-2-aminocyclopentyl]-2-naphtylsulfonamid, N-[(1R,2R) und (1S,2S)-2-aminocyclopentyl]-pentafluorphenylsulfonamid, N-[(1R,2R) und (1S,2S)-2-aminocyclopentyl]-methansulfonamid, N-[(1R,2R) und (1S,2S)-2-aminocyclopentyl]-trifluormethansulfonamid.

In der Formel (IV)

[RuX₂(aren){(III)}] (IV)

besitzen Aren und X jeweils die unter Formel (II) angegebene Bedeutung und Vorzugsbereiche, (III) steht in der Formel (IV) für Verbindungen der Formel (III) mit den dort angegebenen Bedeutung und Vorzugsbereichen.

Als Verbindungen der Formel (IV) seien genannt:
[N-[(1R,2R und 1S,2S)-2-(amino-κN)-1,2-diphenylethyl]-p-tolylsulfonamidato-κN]-chloro[(η⁶)-cumol]-ruthenium(II)
[N-[(1R,2R und 1S,2S)-2-(amino-κN)-1,2-diphenylethyl]-o-tolylsulfonamidato-κN]-chloro[(η⁶)-cumol]-ruthenium(II)
[N-[(1R,2R und 1S,2S)-2-(amino-κN)-1,2-diphenylethyl]-m-tolylsulfonamidato-κN]-chloro[(η⁶)-cumol]-ruthenium(II)
[N-[(1R,2R und 1S,2S)-2-(amino-κN)-1,2-diphenylethyl]-phenylsulfonamidato-κN]-chloro[(η⁶)-cumol]-ruthenium(II)
[N-[(1R,2R und 1S,2S)-2-(amino-κN)-1,2-diphenylethyl]-4-ethylphenylsulfonamidato-κN]chloro[(η⁶)-cumol]-ruthenium(II)
[N-[(1R,2R und 1S,2S)-2-(amino-κN)-1,2-diphenylethyl]-3-ethylphenylsulfonamidato-κN]chloro[(η⁶)-cumol]-ruthenium(II)
[N-[(1R,2R und 1S,2S)-2-(amino-κN)-1,2-diphenylethyl]-2-ethylphenylsulfonamidato-κN]chloro[(η⁶)-cumol]-ruthenium(II)
[N-[(1R,2R und 1S,2S)-2-(amino-κN)-1,2-diphenylethyl]-2,4,6-trimethylphenylsulfonamidato-κN]chloro[(η⁶)-cumol]-ruthenium(II)
[N-[(1R,2R und 1S,2S)-2-(amino-κN)-1,2-diphenylethyl]-2,4,6-triisopropylphenylsulfonamidato-κN]chloro[(η⁶)-cumol]-ruthenium(II)
[N-[(1R,2R und 1S,2S)-2-(amino-κN)-1,2-diphenylethyl]-4-chlorphenylsulfonamidato-κN]chloro[(η⁶)-cumol]-ruthenium(II)
[N-[(1R,2R und 1S,2S)-2-(amino-κN)-1,2-diphenylethyl]-3-chlorphenylsulfonamidato-κN]chloro[(η⁶)-cumol]-ruthenium(II)
[N-[(1R,2R und 1S,2S)-2-(amino-κN)-1,2-diphenylethyl]-2-chlorphenylsulfonamidato-κN]chloro[(η⁶)-cumol]-ruthenium(II)
[N-[(1R,2R und 1S,2S)-2-(amino-κN)-1,2-diphenylethyl]-4-fluorphenylsulfonamidato-κN]chloro[(η⁶)-cumol]-ruthenium(II)
[N-[(1R,2R und 1S,2S)-2-(amino-κN)-1,2-diphenylethyl]-3-fluorphenylsulfonamidato-κN]chloro[(η⁶)-cumol]-ruthenium(II)
[N-[(1R,2R und 1S,2S)-2-(amino-κN)-1,2-diphenylethyl]-2-fluorphenylsulfonamidato-κN]chloro[(η⁶)-cumol]-ruthenium(II)
[N-[(1R,2R und 1 S,2S)-2-(amino-κN)-1,2-diphenylethyl]-4-methoxyphenylsulfonamidato-κN]chloro[(η⁶)-cumol]-ruthenium(II)
[N-[(1R,2R und 1S,2S)-2-(amino-κN)-1,2-diphenylethyl]-3-methoxyphenylsulfonamidato-κN]chloro[(η⁶)-cumol]-ruthenium(II)
[N-[(1R,2R und 1S,2S)-2-(amino-κN)-1,2-diphenylethyl]-2-methoxyphenylsulfonamidato-κN]chloro[(η⁶)-cumol]-ruthenium(II)ato-κN]chloro[(η⁶)-cumol]-ruthenium-(II)
[N-[(1R,2R und 1S,2S)-2-(amino-κN)-1,2-diphenylethyl]-1-naphtylsulfonamidato-κN]chloro[(η⁶)-cumol]-ruthenium(II)
[N-[(1R,2R und 1S,2S)-2-(amino-κN)-1,2-diphenylethyl]-2-naphtylsulfonamidato-κN]chloro[(η⁶)-cumol]-ruthenium(II)
[N-[(1R,2R und 1S,2S)-2-(amino-κN)-1,2-diphenylethyl]-pentafluorphenylsulfonamidato-κN]chloro[(η⁶)-cumol]-ruthenium(II)
[N-[(1R,2R und 1S,2S)-2-(amino-κN)-1,2-diplienylethyl]-methansulfonamidato-κN]-chloro[(η⁶)-cumol]-ruthenium(II)
[N-[(1R,2R und 1S,2S)-2-(amino-κN)-1,2-diphenylethyl]-trifluormethansulfonamidato-κN]chloro[(η⁶)-cumol]-ruthenium(II)
[N-[(1R,2R und 1S,2S)-2-(amino-κN)-1,2-diphenylethyl]-p-tolylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-o-tolylsulibnamidato-κN]-chloro[(η⁶)-1,3,5-trimethylbenzol]-ruthenium(II)
[N-[(1R,2R und 1S,2S)-2-(amino-κN)-1,2-diphenylethyl]-m-tolylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-phenylsulfonamidato-κN]-chloro[(η⁶)-1,3,5-trimethylbenzol]-ruthenium(II)
[N-[(1R,2R und 1S,2S)-2-(ammo-κN)-1,2-diphenylethyl]-4-ethylphcnylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-3-ethylphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-ethylphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2,4,6-trimethylphenylsulfonamidato-κN]chloro[(η⁶)-1,3,5-trimethylbenzol]-ruthenium(II)
[N-[(1R,2R und 1S,2S)-2-(ammo-κN)-1,2-diphenylethyl]-2,4,6-triisopropylphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-4-chlorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-3-chlorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-chlorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-4-fluorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-3-fluorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-fluorphenylsulfonamidato-κN]chloro[(η⁶)-1,3,5-trimethylbenzol]-rthenium(II)
[N-[(1R,2R und 1S,2S)-2-(amino-κN)-1,2-diphenylethyl]-4-methoxyphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-3-methoxyphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-methoxyphenylsulfonamidato-κN]chloro[(η⁶)-1,3,5-trimethylbenzol]-ruthenium(II)ato-kN]chloro[(η⁶)-1,3,5-tnmethylbenzol]-ruthenium(II)
[N-[(1R,2R und 1S,2S)-2-(amino-κN)-1,2-diphenylethyl]-1-naphtylsulfonamidato-[N]chloro[(η⁶)-1,3,5-trimethylbenzol]-ruthenium(II)
[N-[(1R,2R und 1S,2S)-2-(amino-κN)-1,2-diphenylethyl]-2-naphtylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-pentafluorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-methansulfonamidato-κN]-chloro[(η⁶)-1,3,5-trimethylbenzol]-ruthenium(II)
[N-[(1R,2R und 1S,2S)-2-(amino-κN)-1,2-diphenylethyl]-trifluormethansulfonamidato-κN]chloro[(η⁶)-1,3,5-trimethylbenzol]-ruthenium(II)
[N-[(1R,2R und 1S,2S)-2-(amino-κN)-1,2-diphenylethyl]-p-tolylsulfonamidato-κN]-chloro[(η⁶)-benzol]-ruthenium(II)
[N-[(1R,2R und 1S,2S)-2-(amino-κN)-1,2-diphenylethyl]-o-tolysulfonamidato-κN]-chloro[(η⁶)-benzol]-ruthenium(II)
[N-[(1R,2R und 1S,2S)-2-(amino-κN)-1,2-diphenylethyl]-m-tolylsulfonamidato-κN]-chloro[(η⁶)-benzol]-ruthenium(II)
[N-[(1R,2R und 1S,2S)-2-(amino-κN)-1,2-diphenylethyl]-phenylsulfonamidato-κN]-chloro[(η⁶)-benzol]-ruthenium(II)
[N-[(1R,2R und 1S,2S)-2-(amino-κN)-1,2-diphenylethyl]-4-ethylphenylsulfonamidato-κN]chloro[(η⁶)-benzol]-ruthenium(II)
[N-[(1R,2R und 1S,2S)-2-(amino-κN)-1,2-diphenylethyl]-3-ethylphenylsulfonamidato-κN]chloro[(η⁶)-benzol]-ruthenium(II)
[N-[(1R,2R und 1S,2S)-2-(amino-κN)-1,2-diphenylethyl]-2-ethylphenylsulfonamidato-κN]chloro[(η⁶)-benzol]-ruthenium(II)
[N-[(1R,2R und 1S,2S)-2-(amino-κN)-1,2-diphenylethyl]-2,4,6-trimethylphenylsulfonamidato-κN]chloro[(η⁶)-benzol]-ruthenium(II)
[N-[(1R,2R und 1S,2S)-2-(amino-κN)-1,2-diphenylethyl]-2,4,6-triisopropylphenylsulfonamidato-κN]chloro[(η⁶)-benzol]-ruthenium(II)
[N-[(1R,2R und 1S,2S)-2-(amino-κN)-1,2-diphenylethyl]-4-chlorphenylsulfonamidato-κN]chloro[(η⁶)-benzol]-ruthenium(II)
[N-[(1R,2R und 1S,2S)-2-(amino-κN)-1,2-diphenylethyl]-3-chlorphenylsulfonamidato-κN]chloro[(η⁶)-benzol]-ruthenium(II)
[N-[(1R,2R und 1S,2S)-2-(amino-κN)-1,2-diphenylethyl]-2-chlorphenylsulfonamidato-κN]chloro[(η⁶)-benzol]-ruthenium(II)
[N-[(1R,2R und 1S,2S)-2-(amino-κN)-1,2-diphenylethyl]-4-fluorphenylsulfonamidato-κN]chloro[(η⁶)-benzol]-ruthenium(II)
[N-[(1R,2R und 1S,2S)-2-(amino-κN)-1,2-diphenylethyl]-3-fluorphenylsulfonami-κN]chloro[(η⁶)-benzol]-ruthenium(II)
[N-[(1R,2R und 1S,2S)-2-(amino-κN)-1,2-diphenylethyl]-2-fluorphenylsulfonamidato-κN]chloro[(η⁶)-benzol]-ruthenium(II)
[N-[(1R,2R und 1S,2S)-2-(amino-κN)-1,2-diphenylethyl]-4-methoxyphenylsulfonamidato-κN]chloro[(η⁶)-benzol]-ruthenium(II)
[N-[(1R,2R und 1S,2S)-2-(amino-κN)-1,2-diphenylethyl]-3-methoxyphenylsulfonamidato-κN]chloro[(η⁶)-benzol]-ruthenium(II)
[N-[(1R,2R und 1S,2S)-2-(amino-κN)-1,2-diphenylethyl]-2-methoxyphenylsulfonamidato-κN]chloro[(η⁶)-benzol]-ruthenium(II)ato-κN]chloro[(η⁶)-benzol]-ruthenium(II)
[N-[(1R,2R und 1S,2S)-2-(amino-κN)-1,2-diphenylethyl]-1-naphtylsulfonamidato-κN]chloro[(η⁶)-benzol]-ruthenium(II)
[N-[(1R,2R und 1S,2S)-2-(amino-κN)-1,2-diphenylethyl]-2-naphtylsulfonamidato-κN]chloro[(η⁶)-benzol]-ruthenium(II)
[N-[(1R,2R und 1S,2S)-2-(amino-κN)-1,2-diphenylethyl]-pentafluorphenylsulfonamidato-κN]chloro[(η⁶)-benzol]-ruthenium(II)
[N-[(1R,2R und 1S,2S)-2-(amino-κN)-1,2-diphenylethyl]-methansulfonamidato-κN]-chloro[(η⁶)-benzol]-ruthenium(II)
[N-[(1R,2R und 1S,2S)-2-(amino-κN)-1,2-diphenylethyl)-trifluormethansulfonamidato-κN]chloro[(η⁶)-benzol]-ruthenium(II)
[N-[(1R,2R und 1S,2S)-2-(amino-κN)-cyclohexyl]-p-tolylsulfonamidato-κN]chloro-[(η⁶)-benzol]-ruthenium(II)
[N-[(1R,2R und 1S,2S)-2-(amino-κN)-cyclohexyl]-m-tolylsulfonamidato-κN]chloro-[(η⁶)-benzol]-ruthenium(II)
[N-[(1R,2R und 1S,2S)-2-(amino-κN)-cyclohexyl]-phenylsulfonamidato-κN]chloro-[(η⁶)-benzol]-ruthenium(II)
[N-[(1R,2R und 1S,2S)-2-(amino-κN)-cyclohexyl]-4-ethylphenylsulfonamidato-κN]-chloro[(η⁶)-benzol]-ruthenium(II)
[N-[(1R,2R und 1S,2S)-2-(amino-κN)-cyclohexyl]-2,4,6-trimethylphenylsulfonamidato-κN]chloro[(η⁶)-benzol]-ruthenium(II)
[N-[(1R,2R und 1S,2S)-2-(amino-κN)-cyclohexyl]-4-chlorphenylsulfonamidato-κN]-chloro[(η⁶)-benzol]-ruthenium(II)
[N-[(1R,2R und 1S,2S)-2-(amino-κN)-cyclohexyl]-3-chlorphenylsulfonamidato-κN]-chloro[(η⁶)-benzol]-ruthenium(II)
[N-[(1R,2R und 1S,2S)-2-(amino-κN)-cyclohexyl]-4-fluorphenylsulfonamidato-κN]-chloro[(η⁶)-benzol]-ruthenium(II)
[N-[(1R,2R und 1S,2S)-2-(amino-κN)-cyclohexyl]-3-fluorphenylsulfonamidato-κN]-chloro[(η⁶)-benzol]-ruthenium(II)
[N-[(1R,2R und 1S,2S)-2-(amino-κN)-cyclohexyl]-4-methoxyphenylsulfonamidato-κN]chloro[(η⁶)-benzol]-ruthenium(II)
[N-[(1R,2R und 1S,2S)-2-(amino-κN)-cyclohexyl]-3-methoxyphenylsulfonamidato-κN]chloro[(η⁶)-benzol]-ruthenium(II)
[N-[(1R,2R und 1S,2S)-2-(amino-κN)-cyclohexyl]-1-naphtylsulfonamidato-κN] [(η⁶)-benzol]-ruthenium(II)
[N-[(1R,2R und 1S,2S)-2-(amino-κN)-cyclohexyl]-2-naphtylsulfonamidato-κN]-chloro[(η⁶)-benzol]-ruthenium(II)
[N-[(1R,2R und 1S,2S)-2-(amino-κN)-cyclohexyl]-methansulfonamidato-κN]chloro-[(η⁶)-benzol]-ruthenium(II)
[N-[(1R,2R und 1S,2S)-2-(amino-κN)-cyclohexyl]-trifluormethansulfonamidato-κN]chloro[(η⁶)-benzol]-ruthenium(II)
[N-[(1R,2R und 1S,2S)-2-(amino-κN)-cyclohexyl]-p-tolylsulfonamidato-κN]-chloro[(η⁶)-cumol]-ruthenium(II)
[N-[(1R,2R und 1S,2S)-2-(amino-κN)-cyclohexyl]-m-tolylsulfonamidato-κN]-chloro[(η⁶)-cumol]-ruthenium(II)
[N-[(1R,2R und 1S,2S)-2-(amino-κN)-cyclohexyl]-phenylsulfonamidato-κN]-chloro[(η⁶)-cumol]-ruthenium(II)
[N-[(1R,2R und 1S,2S)-2-(amino-κN)-cyclohexyl]-4-ethylphenylsulfonamidato-κN]-chloro[(η⁶)-cumol]-ruthenium(II)
[N-[(1R,2R und 1S,2S)-2-(amino-κN)-cyclohexyl]-2,4,6-trimethylphenylsulfonamidato-κN]chloro[(η⁶)-cumol]-ruthenium(II)
[N-[(1R,2R und 1S,2S)-2-(amino-κN)-cyclohexyl]-4-chlorphenylsulfonamidato-κN]-chloro[(η⁶)-cumol]-ruthenium(II)
[N-[(1R,2R und 1S,2S)-2-(amino-κN)-cyclohexyl]-3-chlorphenylsulfonamidato-κN]-chloro[(η⁶)-cumol]-ruthenium(II)
[N-[(1R,2R und 1S,2S)-2-(amino-κN)-cyclohexyl]-4-fluorphenylsulfonamidato-κN]-chloro[(η⁶)-cumol]-ruthenium(II)
[N-[(1R,2R und 1S,2S)-2-(amino-κN)-cyclohexyl]-3-fluorphenylsulfonamidato-κN]-chloro[(η⁶)-cumol]-ruthenium(II)
[N-[(1R,2R und 1S,2S)-2-(amino-κN)-cyclohexyl]-4-methoxyphenylsulfonamidato-κN]chloro[(η⁶)-cumol]-ruthenium(II)
[N-[(1R,2R und 1S,2S)-2-(amino-κN)-cyclohexyl]-3-methoxyphenylsulfonamidato-κN]chloro[(η⁶)-cumol]-ruthenium(II)
[N-[(1R,2R und 1S,2S)-2-(amino-κN)-cyclohexyl]-1-naphtylsulfonamidato-κN]-chloro[(η⁶)-cumol]-ruthenium(II)
[N-[(1R,2R und 1S,2S)-2-(amino-κN)-cyclohexyl]-2-naphtylsulfonamidato-κN]-chloro[(η⁶)-cumol]-ruthenium(II)
[N-[(1R,2R und 1S,2S)-2-(amino-κN)-cyclohexyl]-methansulfonamidato-κN]chloro-[(η⁶)-cumol]-ruthenium(II)
[N-[(1R,2R und 1S,2S)-2-(amino-κN)-cyclohexyl]-trifluormethansulfonamidato-κN]chloro[(η⁶)-cumol]-ruthenium(II)
[N-[(1R,2R und 1S,2S)-2-(amino-κN)-cyclohexyl]-p-tolylsulfonamidato-κN]chloro-[(η⁶)-1,3,5-trimethylbenzol]-ruthenium(II)
[N-[(1R,2R und 1S,2S)-2-(amino-κN)-cyclohexyl]-m-tolylsulfonamidato-κN]chloro-[(η⁶)-1,3,5-trimethylbenzol]-ruthenium(II)
[N-[(1R,2R und 1S,2S)-2-(amino-κN)-cyclohexyl]-phenylsulfonamidato-κN]chloro-[(η⁶)-1,3,5-trimethylbenzol]-ruthenium(II)
[N-[(1R,2R und 1S,2S)-2-(amino-κN)-cyclohexyl]-4-ethylphenylsulfonamidato-κN]-chloro[(η⁶)-1,3,5-trimethylbenzol]-ruthenium(II)
[N-[(1R,2R und 1S,2S)-2-(amino-κN)-cyclohexyl]-2,4,6-trimethylphenylsulfonamidato-κN]chloro[(η⁶)-1,3,5-trimethylbenzol]-ruthenium(II)
[N-[(1R,2R und 1S,2S)-2-(amino-κN)-cyclohexyl]-4-chlorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-3-chlorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-4-fluorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-3-fluorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-4-methoxyphenylsulfonamidato-κN]chloro[(η⁶)-1,3,5-trimetliylbenzol]-ruthenium(II)
[N-[(1R,2R und 1S,2S)-2-(amino-κN)-cyclohexyl]-3-methoxyphenylsulfonamidato-κN]chloro[(η⁶)-1,3,5-trimethylbenzol]-ruthenium(II)
[N-[(1R,2R und 1S,2S)-2-(amino-κN)-cyclohexyl]-1-naphtylsulfonamidato-κN]-chloro[(η⁶)-1,3,5-trimethylbenzol]-ruthenium(II)
[N-[(1R,2R und 1S,2S)-2-(amino-κN)-cyclohexyl]-2-naphtylsulfonamidato-κN]-chloro[(η⁶)-1,3,5-trimethylbenzol]-ruthenium(II)
[N-[(1R,2R und 1S,2S)-2-(amino-κN)-cyclohexyl]-methansulfonamidato-κN]chloro-[(η⁶)-1,3,5-trimethylbenzol]-ruthenium(II) und
[N-[(1R,2R und 1S,2S)-2-(amino-κN)-cyclohexyl]-trifluormethansulfonamidato-κN]chloro[(η⁶)-1,3,5-trimethylbenzol]-ruthenium(II).

Besonders bevorzugte Katalysatoren im Sinne der Erfindung sind solche, die Rutheniumkomplexe enthalten, die durch Umsetzung von S,S- oder R,R-N-p-Toluolsulfonyl-1,2-diphenylethylendiamin und Cumoldichlororuthenium-Dimer erhältlich sind.

Das erfindungsgemäße Verfahren wird in Gegenwart von mindestens einem Amin, bevorzugt einem Amin durchgeführt, das zumindest teilweise in protonierter Form vorliegt.

Weiterhin werden für das erfindungsgemäße Verfahren Ameisensäure, Formiate oder Mischungen davon eingesetzt.

Bevorzugt werden Mischungen von Ameisensäure mit Aminen eingesetzt. Auf diese Weise bilden sich zumindest teilweise die entsprechenden Ammoniumformiate, die analog eingesetzt werden können.

Als Amine sind insbesondere solche der Formel (V) geeignet

NR⁶R⁷R⁸ (V),

in der
- R⁶, R⁷ und R⁸: jeweils unabhängig voneinander für Wasserstoff, C₁-C₈-Alkyl oder Benzyl stehen.

Besonders bevorzugte Amine sind Ammoniak und solche der Formel (V) in der R⁶, R⁷ und R⁸ jeweils unabhängig voneinander für C₁-C₈-Alkyl oder Benzyl stehen.

Besonders bevorzugte Amine sind solche der Formel (V) in der R⁶, R⁷ und R⁸ jeweils identisch für Ethyl, n-Butyl oder n-Hexyl stehen, wobei der Einsatz von Triethylamin noch weiter bevorzugt ist.

Das molare Verhältnis von Ameisensäure zu tertiärem Amin kann beispielsweise 1:1 bis 3:1 betragen; bevorzugt ist ein Verhältnis von 1,01:1 bis 1,5:1.

Das molare Verhältnis an Ameisensäure bezogen auf eingesetztes Substrat kann beispielsweise 1:1 bis 3:1.betragen, bevorzugt sind 1:1 bis 1,5 : 1, besonders bevorzugt 1,02 : 1 bis 1,1 : 1.

Das erfindungsgemäße Verfahren kann in Gegenwart oder Abwesenheit, bevorzugt in Gegenwart von organischem Lösungsmittel durchgeführt werden.

Geeignete organische Lösungsmittel sind beispielsweise:
Amide wie z.B. Dimethylformamid, N-Methylpyrrolidinon, gegebenenfalls halogenierte aliphatische oder araliphatische Lösungsmittel mit bis zu 16 Kohlenstoffatomen wie z.B. Toluol, o-, m-, p-Xylol, Chloroform, Dichlormethan, Chlorbenzol, die isomeren Dichlorbenzole, Fluorbenzol, Nitrile wie zum Beispiel Acetonitril, Benzonitril, Dimethylsulfoxid oder Mischungen davon.

Bevorzugte Lösungsmittel sind Acetonitril, N-Methylpyrrolidinon, Chloroform, Dichlormethan, Chlorbenzol, die isomeren Dichlorbenzole, Fluorbenzol oder Mischungen davon, besonders bevorzugt sind Dichlormethan, Acetonitril, N-Methylpyrrolidinon oder Mischungen davon.

Die Reaktionstemperatur kann beispielsweise -10 bis 150°C betragen, bevorzugt sind 20 bis 100°C, besonders bevorzugt 20 bis 80°C.

Die Reaktionszeiten liegen beispielsweise zwischen 0,5 h und 48 h, bevorzugt zwischen 6 und 24 h.

Die molare Menge an Ruthenium kann beispielsweise 0,01 bis 1,0 mol-% bezogen auf das eingesetzte Substrat betragen, bevorzugt sind 0,02 bis 0,2 mol-%, ganz besonders bevorzugt 0,02 bis 0,1 mol-%.

Es ist vorteilhaft, wenn auch nicht obligatorisch, die Reaktion in einer im Wesentlichen sauerstofffreien Atmosphäre durchzuführen. Im Wesentlichen sauerstofffrei bedeutet dabei beispielsweise einen Gehalt von 0 bis 1 Vol-%, bevorzugt 0 bis 0,1 Vol-% Sauerstoff.

Die Reaktion kann durch die Entfernung von Kohlendioxid, welches während der Reaktion freigesetzt wird, beschleunigt werden. Vorteilhaft und daher von der Erfindung umfasst ist intensives Rühren des Reaktionsgemisches mit einer durchschnittlichen Rührerdrehzahl von beispielsweise 100 bis 3 000 min⁻¹, bevorzugt 500 bis 1 500 min⁻¹. Alternativ oder in Ergänzung dazu kann die Entfernung von Kohlendioxid durch Durchleiten oder Überleiten eines inerten Gasstroms durch oder über die Reaktionsmischung unterstützt werden. Geeignete Gase sind beispielsweise Stickstoff, Edelgase wie z.B. Argon oder Mischungen davon.

Auf erfindungsgemäße Weise erhält man stereoisomerenangereicherte 3-Heteroaryl-3-rhydroxy-propionsäuredenvate der Formel (VI)

Heteroaryl-CH(OH)-CH₂W (VI),

in der Heteroaryl und W die gleichen Bedeutungen und Vorzugsbereiche besitzen, die unter der Formel (I) genannt wurden.

Dabei sind je nach Wahl der Konfiguration der Liganden die an 3-Position S- oder R-konfigurierten Produkte erhältlich.

Die erfindungsgemäß herstellbaren, stereoisomerenangereicherten 3-Heteroaryl-3-hydroxy-propionsäurederivate eignen sich insbesondere für den Einsatz in einem Verfahren zur Herstellung von flüssigkristallinen Verbindungen, Agrarchemikalien und Pharmazeutika oder Zwischenprodukten davon.

Eine besonders bevorzugte Ausführung des erfindungsgemäßen Prozesses wird nachfolgend beschrieben, ohne jedoch einschränkend zu sein.

In einem Rührkessel wird eine 1:1 Mischung (molar) aus Ameisensäure und Triethylamin durch einfaches Mischen hergestellt und das 3-Heteroaryl-3-oxopropionsäurederivat äquimolar oder in geringem Unterschuss zu dieser zweiphasigen Mischung gegeben. Je nach Löslichkeit des Substrates wird eine Menge eines organischen Lösungsmittels zugegeben. Diese Mischung wird durch Durchleiten von Stickstoff inertisiert und die Mischung unter starkem Rühren auf die gewünschte Reaktionstemperatur temperiert.

Zu dieser Mischung wird der Katalysator als Lösung in Dichlormethan in Molverhältnissen gegenüber dem Substrat von beispielsweise 1:500 bis 1:5 000 gegeben und die Reaktionsmischung die gewünschte Zeit gerührt. Der Umsatz wird chromatographisch verfolgt.

Anschließend kann die Reaktionsmischung nach dem Fachmann bekannten Verfahren aufgearbeitet werden. Es hat sich als vorteilhaft erwiesen, zur Aufarbeitung der Reaktionsmischung Lösungsmittel und verdünnte wässrige Salzsäure oder Wasser zuzusetzen. Nach Phasentrennung kann das Produkt aus der organischen Phase entweder destillativ oder durch ein geeignetes Kristallisationsverfahren in an sich bekannter Weise isoliert werden.

Der Vorteil der vorliegenden Erfindung ist, dass 3-Heteroaryl-3-hydroxypropionsäurederivate in effizienter und technisch einfach durchführbarer Weise stereoisomerenangereichert erhalten werden können, wobei hohe Umsatzzahlen erreicht werden.

### Beispiele

### Allgemeine Arbeitsvorschrift für die Transferhydrierung von 3-Heteroaryl-3-oxopropionsäurederivaten

### (Beispiele 1-23)

In einem Schlenkgefäß wird die Katalysatorlösung durch Einwaage von 2,03 Mol - Äquivalenten 1S,2S-N-(p-Toluolsulfonyl)-1,2-diphenylethylen-diamin (S,S-TsDPEN) und 1 Mol-Äquivalent [(Cumol)RuCl₂]₂, Rühren dieses Gemisches in 5 ml CH₂Cl₂ und Versetzen mit 2 Mol-Äquivalenten Et₃N für 15 min hergestellt.

In einem 100 ml-Mehrhalskolben mit Begasungs-Rührer, Rückflusskühler und Thermometer wird eine Ameisensäure/Et₃N-Mischung (Molverhältnis 1:1, Molverhältnis 1,05:1 bezogen auf das Substrat) durch langsames Zutropfen innerhalb von 5 min per Tropftrichter von HCOOH zum Et₃N unter Rührung und Eiskühlung hergestellt. Zu diesem zweiphasigen Gemisch wird der entsprechende Ketoverbindung gegeben (500 bis 5 000 eq. bezogen auf den Katalysator), die homogene gelbe Lösung gegebenenfalls mit Lösungsmittel versetzt und die Gesamtmischung durch Durchleiten von Argon für 20 min. entgast. Es wird auf Solltemperatur temperiert und unter starkem Rühren die dunkelrote Katalysatorlösung auf einmal zum Reaktionsansatz per Spritze geben. Es wird unter Argon für die gegebene Zeit gerührt.

Es wird mit Wasser und CH₂Cl₂ verdünnt, für 10 min nachgerührt, nach Phasentrennung die H₂O Phase zweimal mit CH₂Cl₂ extrahiert. Die vereinigten organischen Phasen werden mit NaCl-Lösung gewaschen, über MgSO₄ getrocknet, filtriert und dann das Lösungsmittel am Rotationsverdampfer entfernt. Das Rohprodukt wird entweder destilliert, umkristallisiert z.B. aus Hexan/Petrolether bzw. aus Hexan/Dichlormethan oder als Rohmischung in weiteren Reaktionen eingesetzt. Man erhält das Produkt in 90 bis 100 % Ausbeute.

Die Umsatz- und Enantiomeren-Analytik erfolgte gaschromatographisch.
Methyl-3-hydroxy-3-(4-pyridinyl)propanoat (1)
¹H-NMR (d1-Chloroform, 400 MHz): δ = 8.54, 7.35 (je d, je 2H, Py-H, 2J = 6 Hz), 5.12 (dd, 1H, CHOH), 3.69 (s, 3H, OCH3), 2.71 (m, 2H, CHH) ppm.

Chiral-GC: 15.49, 16.07 min.
Ethyl-3-hydroxy-3-(4-pyridinyl)propanoat (2)
¹H-NMR (d1-Chloroform, 400 MHz): δ = 8.54, 7.32 (je d, je 2H, Py-H, 2J = 6 Hz), 5.13 (dd, 1H, CHOH), 4.19 (q, 2H, OCH2), 2.73 (m, 2H, CHH), 1.27 (t, 3H, CH3) ppm.

Chiral-GC: 18.33, 18.60 min.
Ethyl-3-hydroxy-3-(3-pyridinyl)propanoat (3)
¹H-NMR (d1-Chloroform, 400 MHz): δ = 8.62, 8.61, 7.77, 7.33 (je m, je 1H, Py-H), 5.19 (dd, 1H, CHOH), 3.75 (s, 3H, OCH3), 3.44 (d, 1 H, OH), 2.77 (m, 2H, CHH) ppm.

Chiral-GC: 16.69, 17.56 min.
Ethyl(3S)-3-(6-chloro-3-pyridinyl)-3-hydroxypropanoat (4)
¹H-NMR (d1-Chloroform, 400 MHz): δ = 8.32 (d, 2H, Py-H, J = 2 Hz), 7.66 (dd, 2H, Py-H, J = 2 Hz, J = 8 Hz), 7.26 (d, 2H, Py-H, J = 8 Hz), 5.12 (dd, 1H, CHOH), 4.12 (q, 2H, OCH2), 2.68 (m, 2H, CHH), 1.22 (t, 3H, CH3) ppm.

Chiral-GC: 14.12, 14.74 min.
Methyl-3-hydroxy-3-(3-thiophen-yl)propanoat (5)
¹H-NMR (d1-Chloroform, 400 MHz): δ = 7.30, 7.23, 7.08 (je m, je 1H, Ar-H), 5.22 (dd, 1H, CHOH), 3.72 (s, 3H, OCH3), 2.79 (m, 2H, CHH) ppm.

Chiral-GC: 15.56, 15.99 min.
(S)-Methyl-3-hydroxy-3-(2-thiophen-yl)propanoat (6)
¹H-NMR (d1-Chloroform, 400 MHz): δ = 7.23 (m, 1H, Ar-H), 6.95 (m, 2H, Ar-H), 5.36 (dd, 1H, CHOH), 3.71 (s, 3H, OCH3), 2.86 (m, 2H, CHH) ppm.
¹³C-NMR (d1-Chloroform, 100 MHz): δ = 185.3 (C=O), 146.8 (C, Ar), 127.1 (CH, Ar), 125.3 (CH, Ar), 124.1 (CH, Ar), 66.9 (CHOH), 52.4 (CH3), 43.5 (CH2) ppm.

Chiral-GC: 14.05, 14.41 min.
Methyl-3-(3-chloro-1-benzothien-2-yl)-3-hydroxypropanoat (7)
¹H-NMR (d1-Chloroform, 400 MHz): δ = 7.79 (m, 2H, Ar-H), 7.3-7.5 (m, 2H, Ar-H), 5.69 (dd, 1H, CHOH), 3.77 (s, 3H, OCH3) 2.8-3.0 (m, 3H, CHH und OH) ppm.

Chiral-GC: 22.74, 23.47 min.
Methyl-3-hydroxy-3-(3-furanyl)propanoat (8)
¹H-NMR (d1-Chloroform, 400 MHz): δ = 7.42, 7.39, 6.40 (je m, je 1H, Ar-H), 5.09 (dd, 1H, CHOH), 4.19 (q, 2H, OCH2), 3.30 (br, 1H, OH), 2.73 (m, 2H, CHH), 1.27 (t, 3H, CH3) ppm.

Chiral-GC: 5.56, 5.84 min.
Methyl-3-hydroxy-3-(2-furanyl)propanoat (9)
¹H-NMR (d1-Chloroform, 400 MHz): δ = 7.39, 6.34, 6.29 (je m, je 1H, Ar-H), 5.14 (dt, 1H, CHOH), 4.21 (q, 2H, OCH2), 3.24 (d, 1H, OH), 2.90 (dd, 2H, CHH), 2.82 (dd, 2H, CHH), 1.28 (t, 3H, CH3) ppm.

Chiral-GC: 9.02, 9.25 min.
Ethyl-3-hydroxy-3-(2-pyridinyl)propanoat (10)
¹H-NMR (d1-Chloroform, 400 MHz): δ = 8.53, 7.70, 7.42, 7.20 (je m, je 1H, Ar-H), 5.19 (m, 1H, CHOH), 4.18 (q, 2H, OCH2), 2.90 (dd, 1H, CHH), 2.76 (dd, 1H, CHH), 1.25 (t, 3H, CH3) ppm.

Chiral-GC (TMS-Ester): 22.92, 23.52 min.

Die Ergebnisse der Beispiele 1-10 sind in Tabelle 1 zusammengefasst

**Tabelle 1:**

| Beispiel | Zeit [h] | S/C | Umsatz [%] | Enantiomeric excess (ee) [%] |
|---|---|---|---|---|
| 1 | 18 | 500 | 100 | 87,4 |
| 2 | 18 | 500 | 100 | 82,8 |
| 3 | 18 | 500 | 99,2 | 81,9 |
| 4 | 18 | 500 | 100 | 62,5 |
| 5 | 18 | 500 | 100 | 37 |
| 6 | 18 | 500 | 100 | 98,2 (S) |
| 7 | 18 | 500 | 100 | 93,1 |
| 8 | 18 | 500 | 98,7 | 92,3 |
| 9 | 18 | 500 | 99,9 | 96,6 |
| 10 | 18 | 500 | 100 | 88,6 |

Lösungsmittel: CH₂Cl₂, Substrat-Konzentration: 1,6 mol/l, T = 30°C, 1,4 Moläquivalente HCOOH/Et₃N (Molverhältnis 1:1).

### Beispiele 11-15

Lösungsmitteleinfluss auf die Umsatzraten (TOF) und Enantioselektivitäten in der Reduktion von Methyl-3-oxo-3-(2-thiophen-yl)propanoat

Durchführung des Versuches wie in Beispiel 1, aber mit magnetischer Rührung.

Die Ergebnisse der Beispiele 11-15 sind in Tabelle 2 zusammengefasst.

**Tabelle 2:**

| Beispiel | S/C | Lösungsmittel | Mittlere TOF (1 h) | Mittlere TOF (8 h) | Umsatz (16 h) | Ee[%] |
|---|---|---|---|---|---|---|
| 11 | 1000 | CH₂Cl₂ | 185 | 106 | 100 | 97,4 |
| 12 | 1000 | keines | 204 | 108 | 100 | 97,0 |
| 13 | 1000 | NMP | 159 | 112 | 100 | 97,7 |
| 14 | 1000 | CH₃CN | 334 | 115 | 100 | 97,0 |
| 15 | 1000 | DMSO | 152 | 90 | 87,7 | 97,1 |

| | | | | | | |
|---|---|---|---|---|---|---|
| Substrat-Konzentration: 2,2 mol/l, T = 40 °C, 1,3 Moläquivalente HCOOH/Et₃N (Molverhältnis 1:1). | | | | | | |

### Beispiele 16-19

Einfluss der Entfernung von CO₂ auf den Umsatz und die Reaktionsgeschwindigkeit in der Transferhydrierung von Methyl-3-oxo-3-(2-thiophen-yl)propanoat ^{[1]}.

Die Ergebnisse der Beispiele 16-19 sind in Tabelle 3 zusammengefasst.

**Tabelle 3:**

| Beispiel | S/C | Rührung | Begasung | t [h] | U [%] |
|---|---|---|---|---|---|
| 16 | 1500 | Magnetisch | geschlossene Apparatur | 48 | 33 |
| 17 | 1500 | Magnetisch | Überleiten von Ar | 48 | 80 |
| 18 | 1200 | KPG-Rührer | Überleiten von Ar | 17 | 100 |
| 19 | 1200 | KPG-Rührer | Durchleiten von Ar | 12 | 99 |

Lösungsmittel CH₂Cl₂, c = 2,5-2,7 mol/l, T .= 40 °C, 1,07 Moläquivalente HCOOH/Et₃N (Molverhältnis 1:1).

### Beispiele 20-23

Die Beispiele 20-23 wurden analog zu Beispiel 1 durchgeführt.
3-Hydroxy-3-(2-furanyl)propannitril **(20)**
¹H-NMR (D1-Chloroform, 400 MHz): δ = 7.41 (m, 1 H, Ar-H), 6.38, (m, 2H, Ar-H), 5.04 (dd, 1H, CHOH), 3.03 (br, 1H, OH), 2.90 (m, 2H, CHH) ppm.

Chiral-GC: 6.47, 7,29 min. ee = 94.5 %.
(S)-3-Hydroxy-3-(2-thiophen-yl)propannitril **(21)** ¹H-NMR (D1-Chloroform, 400 MHz): δ = 7.32, 7.08, 7.01 (je m, je 1H, Ar-H), 5.28 (dd, 1H, CHOH), 2.86 (m, 3H, CHH und OH) ppm.

Chiral-GC: 13.23, 13.56 min. ee = 97.1%.
3-Hydroxy-3-(3-thiophen-yl)propannitril **(22)**
¹H-NMR (D1-Chloroform, 400 MHz): δ = 7.37, 7.33, 7.12 (je m, je 1H, Ar-H), 5.12 (dd, 1H, CHOH), 2.80 (m, 2H, CHH) 2.75 (br, 1H, OH) ppm.

Chiral-GC: 13.61, 13.97 min. ee = 95.9 %.
3-Hydroxy-3-(6-chloro-3-pyridinyl)propannitril (23)
¹H-NMR (D1-Chloroform, 400 MHz): δ = 8.40 (d, 2H, Py-H, J = 2 Hz), 7.80 (dd, 2H, Py-H, J = 2 Hz, J = 8 Hz), 7.37 (d, 2H, Py-H, J = 8 Hz), 5.14 (dd, 1H, CHOH), 2.82 (m, 2H, CHH) ppm.

Chiral-GC: 24.78, 25.14 min. ee = 73.3 %.

Die Ergebnisse der Beispiele 20-23 sind in Tabelle 4 zusammengefasst.

**Tabelle 4:**

| Beispiel | t [h] | S/C | U [%] | ee [%] |
|---|---|---|---|---|
| 20 | 20 | 250 | 100 | 94,5 |
| 21 | 20 | 250 | 100 | 97,1 |
| 22 | 20 | 250 | 100 | 95,9 |
| 23 | 20 | 250 | 100 | 73,3 |

Lösungsmittel CH₂Cl₂, c= 0,6 mol/l, T = 35 °C, 1,4 Moläquivalente HCOOH/Et₃N (Molverhältnis 1:1).

## Patentansprüche

1. Verfahren zur Herstellung von stereoisomerenangereicherten 3-Heteroaryl-3-hydroxypropionsäurederivaten, **dadurch gekennzeichnet, dass**
a) Verbindungen der Formel (I)
Heteroaryl-CO-CH₂W (I),
in der
Heteroaryl für einen mono-, bi- oder tricyclischen aromatischen Rest mit insgesamt 5 bis 18 Ringatomen steht, wobei pro Cyclus keines, eines zwei oder drei Ringatome und im gesamten aromatischen Rest ein, zwei, drei, vier oder fünf Ringatome ausgewählt aus der Gruppe Sauerstoff, Schwefel und Stickstoff vorhanden sein können, und wobei der mono-, bi- oder tricyclische aromatische Rest gegebenenfalls einfach oder mehrfach substituiert ist und
W für C(O)YRₙ¹ steht, wobei Y für Sauerstoff steht und n = 1 ist oder Y für Stickstoff steht und n = 2 ist, oder
W für CN steht,
R¹ jeweils unabhängig voneinander für Wasserstoff, C₁-C₂₀-Alkyl, C₄-C₁₄-Aryl oder C₅-C₁₅-Arylalkyl oder im Falle, dass Y für Stickstoff steht, die beiden Reste R¹ zusammen für C₃-C₁₂ Alkylen stehen,
b) in Gegenwart eines Ruthenium enthaltenden Katalysators und
c) in Gegenwart von mindestens einem Amin, das zumindest teilweise in protonierter Form vorliegt,
d) mit Ameisensäure, Formiaten oder Mischungen davon
umgesetzt werden.

2. Verfahren gemäß Anspruch 1, **dadurch gekennzeichnet, dass** es in Gegenwart von organischem Lösungsmittel durchgeführt wird.

3. Verfahren gemäß einem oder mehreren der Ansprüche 1 bis 2, **dadurch gekennzeichnet, dass** in Formel (I) Heteroaryl steht für einen mono- oder bicyclischen aromatischen Rest mit insgesamt 5 bis 12 Ringatomen, wobei pro Cyclus keines, eines oder zwei und im gesamten aromatischen Rest ein, zwei, drei oder vier Ringatome ausgewählt aus der Gruppe Sauerstoff, Schwefel und Stickstoff vorhanden sein können und wobei der mono- oder bicyclische aromatische Rest keinen, einen, zwei oder drei Reste pro Cyclus trägt, die jeweils unabhängig voneinander ausgewählt sind aus der Gruppe Hydroxy, C₁-C₁₂-Akyl, Cyano, COOH, COOM, COO-(C₁-C₁₂-Alkyl), COO-(C₄-C₁₀-Aryl), CO-(C₁-C₁₂-Alkyl), CO-(C₄-C₁₀-Aryl), O-(C₁-C₁₂-Alkyl), (C₁-C₁₂-Alkyl)-O-(C₁-C₁₂-Alkyl), (C₄-C₁₀-Aryl)-O-(C₁-C₁₂-Alkyl), O-(C₄-C₁₀-Aryl), O-CO-(C₄-C₁₀-Aryl), O-CO-(C₁-C₁₂-Alkyl), OCOO-(C₁-C₁₂-Alkyl), N-(C₁-C₁₂-Alkyl)₂, NH-(C₁-C₁₂-Akyl), N(C₄-C₁₀-Aryl)₂, NH-(C₄-C₁₀-Aryl), Fluor, Chlor, Brom, Iod, NO₂, SO₃H, SO₃M, SO₂(C₁-C₁₂-Alkyl), SO(C₁-C₁₂-Alkyl), C₁-C₁₂-Fluoralkyl, wobei Fluoralkyl für einen einfach mehrfach oder vollständig durch Fluor substituierten Alkylrest gemäß obiger Definition steht, NHCO-(C₁-C₁₂-Alkyl), CONH₂, CONH-(C₁-C₁₂-Alkyl), NHCOO-(C₁-C₁₂-Alkyl), PO(C₄-C₁₀-Aryl)₂, PO(C₁-C₁₂-Alkyl)₂, PO₃H₂, PO₃M₂, PO₃HM PO(O(C₁-C₁₂-Alkyl)₂, wobei M jeweils für ein Alkalimetallion oder ein halbes Äquivalent eines Erdalkalimetallions steht.

4. Verfahren gemäß einem oder mehreren der Ansprüche 1 bis 2, **dadurch gekennzeichnet, dass** in Formel (I) Heteroaryl steht für 2- oder 3-Thiophen-yl, 2- oder 3-Furan-yl, 2- oder 3-Pyrrolyl, 3- oder 4-Pyrazolyl, 1-, 2-, oder 4-Thiazolyl, 1-, 2-, oder 4-Oxazolyl, 2-, 4- oder 5-Imidazolyl, 2-, 3-, oder 4-Pyridyl, 2- oder 3-Pyrazinyl, 2-, 4-, oder 5-Pyrimidyl, 3-, 4-, 5- oder 6-Pyridazinyl, 2- oder 3-Indolyl, 3-Indazolyl, Indazolyl, 2- oder 3-Benzofuranyl, 2- oder 3-Behzothiophen-yl, 2-, 3- oder 4-Chinolinyl, Isochinolinyl 2-, 4-, 6- oder 7-Pteridinyl oder 2- , 3-, 4-, 5-, 6-, 8-, 9- oder 10-Phenanthrenyl wobei jeder der genannten Reste keinen, einen oder zwei Reste pro Cyclus trägt, die jeweils unabhängig voneinander ausgewählt sind aus der Gruppe C₁-C₄-Alkyl, Cyano, COO-(C₁-C₄-Alkyl), O-(C₁-C₄-Alkyl), N(C₁-C₄-Alkyl)₂, NH-(C₁-C₄-Alkyl), Fluor, Chlor, Brom oder C₁-C₄-Fluoralkyl wie zum Beispiel Trifluormethyl, 2,2,2-Trifluorethyl oder Pentafluorethyl.

5. Verfahren gemäß einem oder mehreren der Ansprüche 1 bis 4, **dadurch gekennzeichnet, dass** in Formel (I) Heteroaryl für 2-Thiophen-yl steht.

6. Verfahren gemäß einem oder mehreren der Ansprüche 1 bis 5, **dadurch gekennzeichnet, dass** in Formel (I) W für COOR¹ steht, wobei R¹ für Wasserstoff oder C₁-C₈-Alkyl steht.

7. Verfahren gemäß einem oder mehreren der Ansprüche 1 bis 5, **dadurch gekennzeichnet, dass** als Verbindungen der Formel (I) Ethyl-3-oxo-3-(2-thiophen-yl)propanoat oder Methyl-3-oxo-3-(2-thiophen-yl)propanoat eingesetzt werden:

8. Verfahren gemäß einem oder mehreren der Ansprüche 1 bis 7, **dadurch gekennzeichnet, dass** als Ruthenium enthaltenden Katalysator solche Katalysatoren verwendet werden, die Rutheniumkomplexe enthalten.

9. Verfahren gemäß Anspruch 8, **dadurch gekennzeichnet, dass** als Rutheniumkomplexe solche der Formel (IV) verwendet werden oder solche, die durch Umsetzung von Verbindungen der Formel (II) mit Verbindungen der Formel (III) erhältlich sind, wobei in den Verbindungen der Formel (II)
[RuX₂(Aren)]₂ (II)
Aren für eine koordinierte aromatische Verbindung mit 6 bis 12 Ring-kohlenstoffatomen, die weiterhin mit bis zu 6 Resten substituiert sein kann, die jeweils unabhängig voneinander ausgewählt sind aus der Gruppe C₁-C₈-Alkyl, Benzyl und Phenyl und
X beispielsweise und bevorzugt für Chlor, Brom oder Iod besonders bevorzugt für Chlor steht
und wobei in der Formel (III) 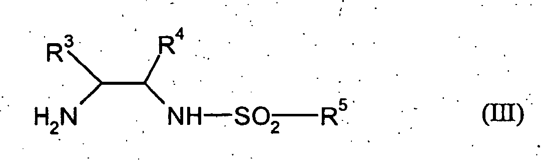
R³ und R⁴ jeweils unabhängig voneinander beispielsweise für C₁-C₂₀-Alkyl, C₄-C₁₅-Aryl oder C₅-C₁₆-Arylalkyl oder R³ und R⁴ zusammen für einen geradkettigen oder verzweigten C₃-C₁₂-Alkylen-Rest, und
R⁵ für C₁-C₂₀-Alkyl, C₁-C₂₀-Fluoralkyl oder C₄-C₁₅-Aryl stehen
und wobei in der Formel (IV)
[RuX₂(aren){(III)}] (IV)
Aren und X jeweils die unter Formel (II) angegebene Bedeutung besitzen und (III) in der Formel (IV) für Verbindungen der Formel (III) mit den dort angegebenen Bedeutung steht.

10. Verfahren gemäß Anspruch 9, **dadurch gekennzeichnet, dass** als Rutheniumkomplexe solche verwendet werden, die durch Umsetzung von Verbindungen der Formel (II) mit Verbindungen der Formel (III) erhältlich sind.

11. Verfahren gemäß einem oder mehreren der Ansprüche 9 bis 10, **dadurch gekennzeichnet, dass** als Rutheniumkomplexe solche verwendet werden, die durch Umsetzung von Verbindungen der Formel (II) mit Verbindungen der Formel (III) erhältlich sind, wobei das molare Verhältnis von Verbindungen der Formel (III) und Verbindungen der Formel (II) 2:1 bis 3:1 beträgt.

12. Verfahren gemäß einem oder mehreren der Ansprüche 9 bis 11, **dadurch gekennzeichnet, dass** als Verbindungen der Formel (II) Benzoldichlororuthenium-Dimer, Mesitylendichlororuthenium-Dimer oder Cumoldichlororuthenium-Dimer eingesetzt werden.

13. Verfahren gemäß einem oder mehreren der Ansprüche 9 bis 12, **dadurch gekennzeichnet, dass** als Verbindungen der Formel (III) solche eingesetzt werden, in denen
R³ und R⁴ jeweils identisch für Phenyl oder zusammen für geradkettiges C₃-C₈-Alkylen stehen und
R⁵ für C₁-C₄-Alkyl, C₁-C₄-Fluoralkyl, Phenyl oder Naphthyl steht, das mit keinem, einem; zwei, drei vier oder fünf Resten weiter substituiert ist, die ausgewählt sind aus der Gruppe C₁-C₄-Alkyl, C₁-C₄-Alkoxy, C₁-C₄-Fluoralkyl, Fluor und Chlor.

14. Verfahren gemäß einem oder mehreren der Ansprüche 9 bis 13, **dadurch gekennzeichnet, dass** als Verbindungen der Formel (III) S,S- oder R,R-N-p-Toluolsulfonyl-1,2-diphenylethylendiamin verwendet werden.

15. Verfahren gemäß einem oder mehreren der Ansprüche 1 bis 14, **dadurch gekennzeichnet, dass** als Amine, die zumindest teilweise in protonierter Form vorliegen, solche der Formel (V) eingesetzt werden,
NR⁶R⁷R⁸ (V)
in der
R⁶, R⁷ und R⁸ jeweils unabhängig voneinander für Wasserstoff, C₁-C₈-Alkyl oder Benzyl stehen.

16. Verfahren gemäß Anspruch 15, **dadurch gekennzeichnet, dass** als Amine, die zumindest teilweise in protonierter Form vorliegen, solche der Formel (V) eingesetzt werden, in der R⁶, R⁷ und R⁸ jeweils unabhängig voneinander für C₁-C₈-Alkyl oder Benzyl stehen.

17. Verfahren gemäß einem oder mehreren der Ansprüche 1 bis 16, **dadurch gekennzeichnet, dass** Mischungen von Ameisensäure und Triethylamin eingesetzt werden.

18. Verfahren gemäß einem oder mehreren der Ansprüche 1 bis 17, **dadurch gekennzeichnet, dass** das molare Verhältnis von Ameisensäure zu Amin 1:1 bis 3:1 beträgt.

19. Verfahren gemäß einem oder mehreren der Ansprüche 1 bis 18, **dadurch gekennzeichnet, dass** das molare Verhältnis an Ameisensäure bezogen auf eingesetztes Substrat 1:1 bis 3:1 beträgt.

20. Verfahren gemäß einem oder mehreren der Ansprüche 1 bis 19, **dadurch gekennzeichnet, dass** die Reaktionstemperatur -10 bis 150°C beträgt.

21. Verfahren gemäß einem oder mehreren der Ansprüche 1 bis 20, **dadurch gekennzeichnet, dass** die molare Menge an Ruthenium 0,01 bis 1,0 mol-%, bezogen auf das eingesetzte Substrat beträgt.

22. Verfahren gemäß einem oder mehreren der Ansprüche 1 bis 21, **dadurch gekennzeichnet, dass** das Reaktionsgemisch mit einer durchschnittlichen Rührerdrehzahl von 100 bis 3 000 min⁻¹ gerührt wird.

23. Verfahren gemäß einem oder mehreren der Ansprüche 1 bis 22, **dadurch gekennzeichnet, dass** durch oder über das Reaktionsgemisch ein inerter Gasstrom geleitet wird.

## Claims

1. Process for preparing stereoisomerically enriched 3-heteroaryl-3-hydroxypropionic acid derivatives, **characterized in that**
a) compounds of the formula (I)
heteroaryl-CO-CH₂W (I)
where
heteroaryl is a mono-, bi- or tricyclic aromatic radical having a total of from 5 to 18 ring atoms where each cycle may have no, one, two or three ring atoms and there may be one, two, three, four or five ring atoms in the entire aromatic radical selected from the group of oxygen, sulphur and nitrogen, and where the mono-, bi- or tricyclic aromatic radical may optionally be mono- or polysubstituted and
W is C(O)YRₙ¹ where Y is oxygen and n = 1 or Y is nitrogen and n = 2, or
W is CN,
R¹ is in each case independently hydrogen, C₁-C₂₀-alkyl, C₄-C₁₄-aryl or C₅-C₁₅-arylalkyl or, in the case that Y is nitrogen, both R¹ radicals together are C₃-C₁₂-alkylene,
b) in the presence of a ruthenium-containing catalyst and
c) in the presence of at least one amine, at least some of which is present in protonated form,
d) are reacted with formic acid, formates or mixtures thereof.

2. Process according to Claim, **characterized in that** it is carried out in the presence of organic solvent.

3. Process according to one or more of Claims 1 to 2, **characterized in that** heteroaryl in formula (I) is a mono- or bicyclic aromatic radical having a total of 5 to 12 ring atoms, where no, one or two, and in the entire aromatic radical, one, two, three or four, ring atoms selected from the group of oxygen, sulphur and nitrogen may be present per cycle, and where the mono- or bicyclic aromatic radical bears no, one, two or three radicals per cycle which are each independently selected from the group of hydroxyl, C₁-C₁₂-alkyl, cyano, COOH, COOM, COO-(C₁-C₁₂-alkyl), COO-(C₄-C₁₀-aryl), CO-(C₁-C₁₂-alkyl), CO-(C₄-C₁₀-aryl), O-(C₁-C₁₂-alkyl), (C₁-C₁₂-alkyl)-O-(C₁-C₁₂-alkyl), (C₄-C₁₀-aryl)-O-(C₁-C₁₂-alkyl), O-(C₄-C₁₀-aryl), O-CO-(C₄-C₁₀-aryl), O-CO-(C₁-C₁₂-alkyl), OCOO-(C₁-C₁₂-alkyl), N-(C₁-C₁₂-alkyl)₂, NH-(C₁-C₁₂-alkyl), N(C₄-C₁₀-aryl)₂, NH-(C₄-C₁₀-aryl), fluorine, chlorine, bromine, iodine, NO₂, SO₃H, SO₃M, SO₂(C₁-C₁₂-alkyl), SO(C₁-C₁₂-alkyl), C₁-C₁₂-fluoroalkyl where fluoroalkyl is a singly, multiply or fully fluorine-substituted alkyl radical as defined above, NHCO-(C₁-C₁₂-alkyl), CONH₂, CONH-(C₁-C₁₂-alkyl), NHCOO-(C₁-C₁₂-alkyl), PO(C₄-C₁₀-aryl)₂, PO(C₁-C₁₂-alkyl)₂, PO₃H₂, PO₃M₂, PO₃HM, PO(O(C₁-C₁₂-alkyl)₂, where M is in each case an alkali metal ion or half an equivalent of an alkaline earth metal ion.

4. Process according to one or more of Claims 1 to 2, **characterized in that** heteroaryl in formula (I) is 2- or 3-thiophenyl, 2- or 3-furanyl, 2- or 3-pyrrolyl, 3- or 4-pyrazolyl, 1-, 2-, or 4-thiazolyl, 1-, 2-, or 4-oxazolyl, 2-, 4- or 5-imidazolyl, 2-, 3-, or 4-pyridyl, 2- or 3-pyrazinyl, 2-, 4-, or 5-pyrimidyl, 3-, 4-, 5- or 6-pyridazinyl, 2- or 3-indolyl, 3-indazolyl, indazolyl, 2- or 3-benzofuranyl, 2- or 3-benzothiophenyl, 2-, 3- or 4-quinolinyl, isoquinolinyl 2-, 4-, 6- or 7-pteridinyl or 2- , 3-, 4-, 5-, 6-, 8-, 9- or 10-phenanthrenyl where each of the radicals mentioned bears no, one or two radicals per cycle, each of which is independently selected from the group of C₁-C₄-alkyl, cyano, COO-(C₁-C₄-alkyl), O-(C₁-C₄-alkyl), N(C₁-C₄-alkyl)₂, NH-(C₁-C₄-alkyl), fluorine, chlorine, bromine or C₁-C₄-fluoroalkyl, for example trifluoromethyl, 2,2,2-trifluoroethyl or pentafluoroethyl.

5. Process according to one or more of Claims 1 to 4, **characterized in that** heteroaryl in formula (I) is 2-thiophenyl.

6. Process according to one or more of Claims 1 to 5, **characterized in that** W in formula (I) is COOR¹ where R¹ is hydrogen or C₁-C₈-alkyl.

7. Process according to one or more of Claims 1 to 5, **characterized in that** the compounds of the formula (I) used are ethyl 3-oxo-3-(2-thiophenyl)-propanoate or methyl 3-oxo-3-(2-thiophenyl)propanoate.

8. Process according to one or more of Claims 1 to 7, **characterized in that** the ruthenium-containing catalyst used is a catalyst which comprises ruthenium complexes.

9. Process according to Claim 8, **characterized in that** the ruthenium complexes used are those of the formula (IV) or those obtainable by reacting compounds of the formula (II) with compounds of the formula (III) where, in the compounds of the formula (II)
[RuX₂(arene)]₂ (II),
arene is a coordinated aromatic compound having 6 to 12 ring carbon atoms which may further be substituted by up to 6 radicals, each of which is independently selected from the group of C₁-C₈-alkyl, benzyl and phenyl and
X is, for example and with preference, chlorine, bromine or iodine, more preferably chlorine,
and where, in the formula (III) 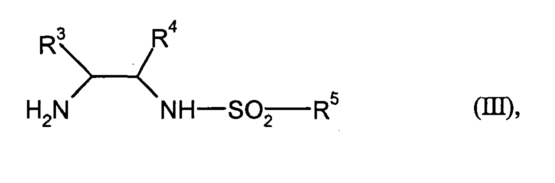
R³ and R⁴ are each independently, for example, C₁-C₂₀-alkyl, C₄-C₁₅-aryl or C₅-C₁₆-arylalkyl, or R³ and R⁴ together are a straight-chain or branched C₃-C₁₂-alkylene radical, and
R⁵ is C₁-C₂₀-alkyl, C₁-C₂₀-fluoroalkyl or C₄-C₁₅-aryl,
and where, in the formula (IV)
[RuX₂(arene){(III)}] (IV),
arene and X are each as defined under formula (II) and (III) in the formula (IV) represents compounds of the formula (III) as defined there.

10. Process according to Claim 9, **characterized in that** the ruthenium complexes used are those obtainable by reacting compounds of the formula (II) with compounds of the formula (III).

11. Process according to one or more of Claims 9 to 10, **characterized in that** the ruthenium complexes used are those obtainable by reacting compounds of the formula (II) with compounds of the formula (III), where the molar ratio of compounds of the formula (III) to compounds of the formula (II) is 2:1 to 3:1.

12. Process according to one or more of Claims 9 to 11, **characterized in that** the compounds of the formula (II) used are the (benzene)dichlororuthenium dimer, the (mesitylene)dichlororuthenium dimer or the (cumene)dichlororuthenium dimer.

13. Process according to one or more of Claims 9 to 12, **characterized in that** the compounds of the formula (III) used are those wherein
R³ and R⁴ are identical and are each phenyl or together are straight-chain C₃-C₈-alkylene and
R⁵ is C₁-C₄-alkyl, C₁-C₄-fluoroalkyl, phenyl or naphthyl which is substituted by none, one, two, three, four or five radicals which are selected from the group of C₁-C₄-alkyl, C₁-C₄-alkoxy, C₁-C₄-fluoroalkyl, fluorine and chlorine.

14. Process according to one or more of Claims 9 to 13, **characterized in that** the compounds of the formula (III) used are S,S- or R,R-N-p-toluenesulphonyl-1,2-diphenylethylenediamine.

15. Process according to one or more of Claims 1 to 14, **characterized in that** the amines used, at least some of which are present in protonated form, are those of the formula (V) where
NR⁶R⁷R⁸ (V)
where
R⁶, R⁷ and R⁸ are each independently hydrogen, C₁-C₈-alkyl or benzyl.

16. Process according to Claim 15, **characterized in that** the amines used, of which at least some are present in protonated form, are those of the formula (V) where R⁶, R⁷ and R⁸ are each independently C₁-C₈-alkyl or benzyl.

17. Process according to one or more of Claims 1 to 16, **characterized in that** mixtures of formic acid and triethylamine are used.

18. Process according to one or more of Claims 1 to 17, **characterized in that** the molar ratio of formic acid to amine is 1:1 to 3:1.

19. Process according to one or more of Claims 1 to 18, **characterized in that** the molar ratio of formic acid based on substrate used is 1:1 to 3:1.

20. Process according to one or more of Claims 1 to 19, **characterized in that** the reaction temperature is -10 to 150°C.

21. Process according to one or more of Claims 1 to 20, **characterized in that** the molar amount of ruthenium is 0.01 to 1.0 mol%, based on the substrate used.

22. Process according to one or more of Claims 1 to 21, **characterized in that** the reaction mixture is stirred at an average stirrer speed of 100 to 3000 min⁻¹.

23. Process according to one or more of Claims 1 to 22, **characterized in that** an inert gas stream is passed through or over the reaction mixture.

## Revendications

1. Procédé de préparation de dérivés d'acide 3-hétéroaryl-3-hydroxypropionique enrichis en stéréoisomère, **caractérisé en ce que** :
a) des composés de formule (I)
hétéroaryl-CO-CH₂W (I)
dans laquelle :
le groupe hétéroaryle représente un radical aromatique monocyclique, bicyclique ou tricyclique renfermant au total de 5 à 18 atomes du noyau, où zéro, un, deux ou trois atomes du noyau peuvent être présents par cycle, et un, deux, trois, quatre ou cinq atomes du noyau peuvent être présents dans le radical aromatique entier, choisis parmi le groupe constitué d'un atome d'oxygène, d'un atome de soufre et d'un atome d'azote, et où le radical aromatique monocyclique, bicyclique ou tricyclique est éventuellement monosubstitué ou polysubstitué, et
W représente un groupe C(O)YRₙ¹, dans lequel Y représente un atome d'oxygène et n vaut 1, ou Y représente un atome d'azote et n vaut 2, ou
W représente un groupe CN,
R¹ représente, dans chaque cas indépendamment, un atome d'hydrogène, un groupe alkyle en C₁-C₂₀, un groupe aryle en C₄-C₁₄ ou un groupe arylalkyle en C₅-C₁₅, ou dans le cas où Y représente un atome d'azote, les deux radicaux R¹ représentent conjointement un groupe alkylène en C₃-C₁₂,
sont mis à réagir,
b) en présence d'un catalyseur renfermant du ruthénium, et
c) en présence d'au moins une amine qui est présente au moins en partie sous la forme protonée,
d) avec de l'acide formique, des formiates ou leurs mélanges.

2. Procédé selon la revendication 1, **caractérisé en ce qu'**il est réalisé en présence d'un solvant organique.

3. Procédé selon l'une ou plusieurs des revendications 1 à 2, **caractérisé en ce que**, dans la formule (I), le groupe hétéroaryle représente un radical aromatique monocyclique ou bicyclique renfermant au total de 5 à 12 atomes du noyau, où zéro, un ou deux atomes du noyau peuvent être présents par cycle, et un, deux, trois ou quatre atomes du noyau peuvent être présents dans le radical aromatique entier, choisis parmi le groupe constitué d'un atome d'oxygène, d'un atome de soufre et d'un atome d'azote, et où le radical aromatique monocyclique ou bicyclique porte un, deux ou trois radicaux par cycle qui sont choisis, dans chaque cas indépendamment les uns des autres, parmi le groupe constitué d'un groupe hydroxyle, d'un groupe alkyle en C₁-C₁₂, d'un groupe cyano, d'un groupe COOH, d'un groupe COOM, d'un groupe COO-(C₁-C₁₂ alkyle), d'un groupe COO-(C₄-C₁₀ aryle), d'un groupe CO-(C₁-C₁₂ alkyle), d'un groupe CO-(C₄-C₁₀ aryle), d'un groupe O-(C₁-C₁₂ alkyle), d'un groupe (C₁-C₁₂ alkyl)-O-(C₁-C₁₂ alkyle), d'un groupe (C₄-C₁₀ aryle)-O-(C₁-C₁₂ alkyle), d'un groupe O-(C₄-C₁₀ aryle), d'un groupe O-CO-(C₄-C₁₀ aryle), d'un groupe O-CO-(C₁-C₁₂ alkyle), d'un groupe OCOO-(C₁-C₁₂ alkyle), d'un groupe N-(C₁-C₁₂ alkyle)₂, d'un groupe NH-(C₁-C₁₂ alkyle), d'un groupe N(C₄-C₁₀ aryle)₂, d'un groupe NH-(C₄-C₁₀ aryle), d'un atome de fluor, d'un atome de chlore, d'un atome de brome, d'un atome d'iode, d'un groupe NO₂; d'un groupe SO₃H, d'un groupe SO₃M, d'un groupe SO₂(C₁-C₁₂ alkyle), d'un groupe SO(C₁-C₁₂ alkyle), d'un groupe fluoroalkyle en C₁-C₁₂, où le groupe fluoroalkyle représente un radical alkyle monosubstitué, polysubstitué ou entièrement substitué par du fluor selon la définition ci-dessus, d'un groupe NHCO-(C₁-C₁₂ alkyle), d'un groupe CONH₂, d'un groupe CONH-(C₁-C₁₂ alkyle), d'un groupe NHCOO-(C₁-C₁₂ alkyle), d'un groupe PO(C₄-C₁₀ aryle)₂, d'un groupe PO(C₁-C₁₂ alkyle)₂, d'un groupe PO₃H₂, d'un groupe PO₃M₂, d'un groupe PO₃HM et d'un groupe PO(O(C₁-C₁₂ alkyle)₂, dans lesquels M représente, dans chaque cas, un ion de métal alcalin ou un demi-équivalent d'un ion de métal alcalino-terreux.

4. Procédé selon l'une ou plusieurs des revendications 1 à 2, **caractérisé en ce que**, dans la formule (I), le groupe hétéroaryle représente un groupe 2- ou 3-thiophényle, un groupe 2- ou 3-furanyle, un groupe 2- ou 3-pyrrolyle, un groupe 3- ou 4-pyrazolyle, un groupe 1-, 2-, ou 4-thiazolyle, un groupe 1-, 2-, ou 4- oxazolyle, un groupe 2-, 4- ou 5-imidazolyle, un groupe 2-, 3- ou 4-pyridyle, un groupe 2- ou 3-pyrazinyle, un groupe 2-, 4- ou 5-pyrimidyle, un groupe 3-, 4-, 5- ou 6-pyridazinyle, un groupe 2- ou 3-indolyle, un groupe 3-indazolyle, un groupe indazolyle, un groupe 2- ou 3-benzofuranyle, un groupe 2- ou 3-benzothiophényle, un groupe 2-, 3- ou 4-quinoléinyle, un groupe isoquinoléinyle, un groupe 2-, 4-, 6- ou 7-ptéridinyle ou un groupe 2-, 3-, 4-, 5-, 6-, 8-, 9- ou 10-phénanthrényle, où chacun des radicaux mentionnés portent zéro, un ou deux radicaux par cycle, qui sont choisis, dans chaque cas indépendamment les uns des autres, parmi le groupe constitué d'un groupe alkyle en C₁-C₄, d'un groupe cyano, d'un groupe COO-(C₁-C₄ alkyle), d'un groupe O-(C₁-C₄ alkyle), d'un groupe N(C₁-C₄ alkyle)₂, d'un groupe NH-(C₁-C₄ alkyle), d'un atome de fluor, d'un atome de chlore, d'un atome de brome ou d'un groupe fluoroalkyle en C₁-C₄ tel que, par exemple, un groupe trifluorométhyle, un groupe 2,2,2-trifluoroéthyle ou un groupe pentafluoroéthyle.

5. Procédé selon l'une ou plusieurs des revendications 1 à 4, **caractérisé en ce que**, dans la formule (I), le groupe hétéroaryle représente un groupe 2-thiophényle.

6. Procédé selon l'une ou plusieurs des revendications 1 à 5, **caractérisé en ce que**, dans la formule (I), W représente un groupe COOR¹, dans lequel R¹ représente un atome d'hydrogène ou un groupe alkyle en C₁-C₈.

7. Procédé selon l'une ou plusieurs des revendications 1 à 5, **caractérisé en ce que**, en tant que composés de formule (I), on utilise l'éthyle 3-oxo-3-(2-thiophényl)propanoate ou le méthyle 3-oxo-3-(2-thiophényl)propanoate.

8. Procédé selon l'une ou plusieurs des revendications 1 à 7, **caractérisé en ce que**, en tant que catalyseur renfermant du ruthénium, on utilise les catalyseurs qui renferment des complexes de ruthénium.

9. Procédé selon la revendication 8, **caractérisé en ce que**, en tant que complexes de ruthénium, on utilise ceux de formule (IV) ou ceux qui peuvent être obtenus par la réaction de composés de formule (II) avec des composés de formule (III), où, dans les composés de formule (II)
[RuX₂(arène)]₂ (II)
le groupe arène représente un composé aromatique coordiné renfermant de 6 à 12 atomes de carbone du noyau, qui peut en outre être substitué par jusqu'à 6 radicaux qui sont choisis, dans chaque cas indépendamment les uns des autres, parmi le groupe constitué d'un groupe alkyle en C₁-C₈, d'un groupe benzyle et d'un groupe phényle, et
X représente, par exemple et de préférence, un atome de chlore, un atome de brome ou un atome d'iode, particulièrement préférablement un atome de chlore,
et où, dans la formule (III) 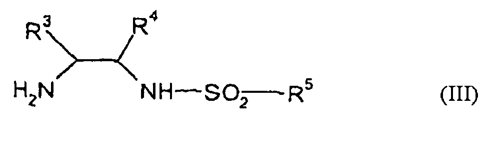
R³ et R⁴ représentent, dans chaque cas indépendamment l'un de l'autre, par exemple, un groupe alkyle en C₁-C₂₀, un groupe aryle en C₄-C₁₅ ou un groupe arylalkyle en C₅-C₁₆, où R³ et R⁴ représentent conjointement un radical alkylène en C₃-C₁₂ linéaire ou ramifié, et
R⁵ représente un groupe alkyle en C₁-C₂₀, un groupe fluoroalkyle en C₁-C₂₀ ou un groupe aryle en C₄-C₁₅,
et où, dans la formule (IV)
[RuX₂(arène) {(III)}] (IV)
le groupe arène et X présentent, dans chaque cas, la signification indiquée pour la formule (II), et (III) dans la formule (IV) représente des composés de formule (III) présentant la signification qui y est indiquée.

10. Procédé selon la revendication 9, **caractérisée en ce que**, en tant que complexes de ruthénium, on utilise ceux qui peuvent être obtenus par la réaction de composés de formule (II) avec des composés de formule (III).

11. Procédé selon l'une ou plusieurs des revendications 9 à 10, **caractérisé en ce que**, en tant que complexes de ruthénium, on utilise ceux qui peuvent être obtenus par la réaction de composés de formule (II) avec des composés de formule (III), où le rapport molaire entre les composés de formule (III) et les composés de formule (II) est de 2:1 à 3:1.

12. Procédé selon l'une ou plusieurs des revendications 9 à 11, **caractérisé en ce que**, en tant que composés de formule (II), on utilise le dimère de benzène-dichlororuthénium, le dimère de mésithylène-dichlororuthénium ou le dimère de cumène-dichlororuthénium.

13. Procédé selon l'une ou plusieurs des revendications 9 à 12, **caractérisé en ce que**, en tant que composés de formule (III), on utilise ceux dans lesquels :
R³ et R⁴ représentent, dans chaque cas identiquement, un groupe phényle, ou représentent conjointement un groupe alkylène en C₃-C₈ linéaire, et
R⁵ représente un groupe alkyle en C₁-C₄, un groupe fluoroalkyle en C₁-C₄, un groupe phényle ou un groupe naphtyle, qui est en outre substitué par zéro, un, deux, trois, quatre ou cinq radicaux qui sont choisis parmi le groupe constitué d'un groupe alkyle en C₁-C₄, d'un groupe alcoxy en C₁-C₄, d'un groupe fluoroalkyle en C₁-C₄, d'un atome de fluor et d'un atome de chlore.

14. Procédé selon l'une ou plusieurs des revendications 9 à 13, **caractérisé en ce que**, en tant que composés de formule (III), on utilise de la S,S- ou de la R,R-N-p-toluènesulfonyl-1,2-diphényléthylènediamine.

15. Procédé selon l'une ou plusieurs des revendications 1 à 14, **caractérisé en ce que**, en tant qu'amines qui sont présentes au moins en partie sous la forme protonée, on utilise celles de formule (V)
NR⁶R⁷R⁸ (V)
dans laquelle :
R⁶, R⁷ et R⁸ représentent, dans chaque cas indépendamment les uns des autres, un atome d'hydrogène, un groupe alkyle en C₁-C₈ ou un groupe benzyle.

16. Procédé selon la revendication 15, **caractérisé en ce que**, en tant qu'amines qui sont présentes au moins en partie sous la forme protonée, on utilise celles de formule (V), dans laquelle R⁶, R⁷ et R⁸ représentent, dans chaque cas indépendamment les uns des autres, un groupe alkyle en C₁-C₈ ou un groupe benzyle.

17. Procédé selon l'une ou plusieurs des revendications 1 à 16, **caractérisé en ce que** l'on utilise des mélanges de l'acide formique et de la triéthylamine.

18. Procédé selon l'une ou plusieurs des revendications 1 à 17, **caractérisé en ce que** le rapport molaire entre l'acide formique et l'amine est de 1:1 à 3:1.

19. Procédé selon l'une ou plusieurs des revendications 1 à 18, **caractérisé en ce que** le rapport molaire de l'acide formique par rapport au substrat utilisé est de 1:1 à 3:1.

20. Procédé selon l'une ou plusieurs des revendications 1 à 19, **caractérisé en ce que** la température de réaction est de -10 à 150°C.

21. Procédé selon l'une ou plusieurs des revendications 1 à 20, **caractérisé en ce que** la quantité molaire de ruthénium est de 0,01 à 1,0 % en moles, par rapport au substrat utilisé.

22. Procédé selon l'une ou plusieurs des revendications 1 à 21, **caractérisé en ce que** le mélange réactionnel est agité avec une vitesse de rotation moyenne de l'agitateur de 100 à 3000 min⁻¹.

23. Procédé selon l'une ou plusieurs des revendications 1 à 22, **caractérisé en ce que** l'on fait passer un courant gazeux inerte à travers ou par-dessus le mélange réactionnel.
